# EUROPÄISCHE PATENTSCHRIFT

(11) **EP 0 805 804 B1**
(45) Veröffentlichungstag und Bekanntmachung des Hinweises auf die Patenterteilung: **29.09.2004**
(21) Anmeldenummer: 96900955.4
(22) Anmeldetag: 15.01.1996
(51) Int. Cl.: C07D 249/12, C07D 249/14, C07D 249/16, C07D 409/12, A01N 43/653, A01N 43/10

(54) **SULFONYLAMINO(THIO)CARBONYL-1,2,4-TRIAZOLIN(THI)ON DERIVATE, DEREN HERSTELLUNG UND DEREN VERWENDUNG ALS HERBIZIDE**
SULPHONYL AMINO(THIO)CARBONYL-1,2,4-TRIAZOLIN(THI)ONE DERIVATIVES, THE PRODUCTION THEREOF AND THE USE THEREOF AS HERBICIDES
DERIVES SULFONYLAMINO(THIO)CARBONYL-1,2,4-TRIAZOLINE(THI)ONES, LEUR PREPARATION ET LEUR UTILISATION COMME HERBICIDES

(30) Priorität: 27.01.1995 DE 19502579
(43) Veröffentlichungstag der Anmeldung: 12.11.1997
(73) Patentinhaber: Bayer CropScience AG, 40789 Monheim (DE)
(72) Erfinder: MÜLLER, Klaus-Helmut, D-40593 Düsseldorf (DE); KLUTH, Joachim, D-40764 Langenfeld (DE); GESING, Ernst, Rudolf, F., D-40699 Erkrath (DE); KÖNIG, Klaus, D-51519 Odenthal (DE); DOLLINGER, Markus, D-51381 Leverkusen (DE); SANTEL, Hans-Joachim, D-51371 Leverkusen (DE)
(86) Internationale Anmeldenummer: PCT/EP1996/000141
(87) Internationale Veröffentlichungsnummer: WO 1996/022982

(56) Entgegenhaltungen:
- EP-A- 0 294 666
- EP-A- 0 341 489
- EP-A- 0 415 196
- EP-A- 0 422 469
- EP-A- 0 425 948
- EP-A- 0 431 291
- EP-A- 0 507 171
- EP-A- 0 534 266
- EP-A- 0 625 515
- ANGEWANDTE CHEMIE, Bd. 81, Nr. 14, Juli 1969 Seiten 534-536, BRUNN E. & HUISGEN R. 'Struktur und Reaktivität des Betains aus Triphenylphosphin und Azodicarbonsäureester'
- CHEMICAL ABSTRACTS, vol. 95, no. 23, 7.Dezember 1981 Columbus, Ohio, US; abstract no. 203841j, RUDNICKA W. ET AL. 'Some derivatives of 4-amino-1,2,4-triazole-3-thione' Seite 673; Spalte 1; & ACTA POL. PHARM. , Bd. 38, Nr. 2, 1981 Seiten 153-162,

## Beschreibung

Die Erfindung betrifft Sulfonylaminothiocarbonyl-triazolinone, welche eine Thiocarbonylgruppe [-C(=S)-] besitzen, mehrere Verfahren zu ihrer Herstellung und ihre Verwendung als Herbizide.

Es ist bekannt, daß bestimmte Sulfonylaminocarbonyltriazolinone herbizide Eigenschaften aufweisen (vgl. EP-A 341489, EP-A 422469, EP-A425948, EP-A 431291, EP-A 507 171, EP-A 534 266). Sulfonylaminothiocarbonyl-triazolinone mit wenigstens einer Thiocarbonylgruppe waren dagegen bisher nicht bekannt.

Es wurden nun die neuen Sulfonylaminothiocarbonyl-triazolinone der allgemeinen Formel (I) 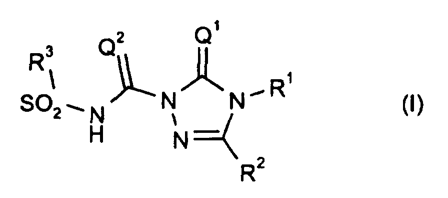 in welcher
- Q¹: für Sauerstoff steht,
- Q²: für Schwefel steht,
- R¹: für Wasserstoff, Hydroxy, Amino, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxy-carbonyl substituiertes C₁-C₆-Alkoxy, C₁-C₆-Alkylamino oder C₁-C₆-Alkanoylamino, für C₃-C₆-Alkenyloxy, für Di-(C₁-C₄-alkyl)-amino, für jeweils gegebenenfalls durch Fluor, Chlor, Brom, Cyano und/oder C₁-C₄-Alkyl substituiertes C₃-C₆-Cycloalkyl, C₃-C₆-Cycloalkylamino oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl und/oder C₁-C₄-Alkoxy substituiertes Phenyl oder Phenyl-C₁-C₄-alkyl steht,
- R²: für Wasserstoff, Hydroxy, Mercapto, Amino, Fluor, Chlor, Brom, Iod, für gegebenenfalls durch Fluor, Chlor, Brom, Cyano, C₁-C₄-Alkoxy, C₁-C₄-Alkylcarbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxy-carbonyl substituiertes C₁-C₆-Alkoxy, C₁-C₆-Alkylthio, C₁-C₆-Alkylamino oder C₁-C₆-Alkanoylamino, für C₃-C₆-Alkenyloxy, C₃-C₆-Alkinyloxy, C₃-C₆-Alkenylthio, C₃-C₆-Alkinylthio, C₃-C₆-Alkenylamino oder C₃-C₆-Alkinylamino, für Di-(C₁-C₄-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes C₃-C₆-Cycloalkyl, C₅-C₆-Cycloalkenyl, C₃-C₆-Cycloalkyloxy, C₃-C₆-Cycloalkylthio, C₃-C₆-Cycloalkylamino, C₃-C₆-Cycloalkyl-C₁-C₄-alkyl, C₃-C₆-Cycloalkyl-C₁-C₄-alkoxy, C₃-C₆-Cycloalkyl-C₁-C₄-alkylthio oder C₃-C₆-Cycloalkyl-C₁-C₄-alkylamino, oder für jeweils gegebenenfalls durch Fluor, Chlor, Brom, Cyano, Nitro, C₁-C₄-Alkyl, Trifluormethyl und/oder C₁-C₄-Alkoxy substituierte substituiertes Phenyl, Phenyl-C₁-C₄-alkyl, Phenoxy, Phenyl-C₁-C₄-alkoxy, Phenylthio, Phenyl-C₁-C₄-alkylthio, Phenylamino oder Phenyl-C₁-C₄-alkylamino steht, oder
- R¹ und R²: zusammen für gegebenenfalls verzweigtes Alkandiyl mit 3 bis 11 Kohlenstoffatomen stehen, und
- R³: für die Gruppierung 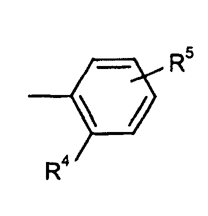 steht, worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylaminocarbonyl, Di-(C₁-C₄-alkyl)-amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₂-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxy-carbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxy-carbonyl, C₁-C₄-Alkylamino-carbonyl oder Di-(C₁-C₄-alkyl)-aminocarbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxy- carbonyl substituiert ist) steht,
R⁴ und/oder R⁵ weiterhin für Phenyl oder Phenoxy, für C₁-C₄-Alkylcarbonylamino, C₁-C₄-Alkoxy-carbonylamino, C₁-C₄-Alkylamino-carbonylamino, Di-(C₁-C₄-alkyl)-amino-carbonylamino, oder für den Rest -CO-R⁸ stehen, wobei
R⁸ für Wasserstoff, C₁-C₆-Alkyl, C₃-C₆-Cycloalkyl, C₁-C₆-Alkoxy, C₃-C₆-Cycloalkoxy, C₃-C₆-Alkenyloxy, C₁-C₄-Alkylthio, C₁-C₄-Alkylamino, C₁-C₄-Alkoxyamino, C₁-C₄-Alkoxy-C₁-C₄-alkylamino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkylsulfonyloxy, Di-(C₁-C₄-alkyl)-aminosulfonylamino oder für den Rest-CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenyloxy, C₃-C₆-Alkinyloxy oder Benzyloxy, für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht,
weiterhin
- R³: für den Rest 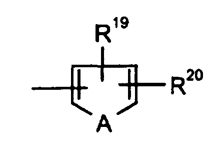 steht, worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), C₁-C₄-Alkylthio, C₁-C₄-Alkylsulfinyl oder C₁-C₄-Alkylsulfonyl (welche jeweils gegebenenfalls durch Fluor und/oder Chlor substituiert sind), Di-(C₁-C₄-alkyl)-aminosulfonyl, C₁-C₄-Alkoxy-carbonyl oder Dimethylaminocarbonyl stehen, und
A für, Schwefel steht,
sowie deren Natrium-, Kalium-, Magnesium-, Calcium-, Ammonium-, C₁-C₄-Alkylammonium-, Di-(C₁-C₄-alkyl)-ammonium-, Tri-(C₁-C₄-alkyl)-ammonium-, Tetra-(C₁-C₄alkyl)-ammonium, Tri-(C₁-C₄-alkyl)-sulfonium-, C₅- oder C₆-Cycloalkyl-ammoniumund Di-(C₁-C₂-alkyl)-benzyl-ammonium-Salze.

Man erhält die neuen Sulfonylaminothiocarbonyl-triazolinthione der allgemeinen Formel (I), wenn man
(a) Triazolinone der allgemeinen Formel (II) 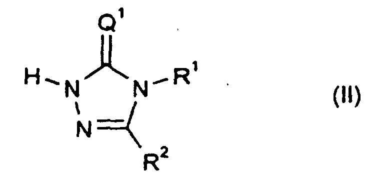 in welcher
   Q¹, R¹ und R² die oben angegebene Bedeutung haben,
   mit Sulfonylisothiocyanaten der allgemeinen Formel (III)

   R³-SO₂-N=C=Q² (III)

   in welcher
   Q² und R³ die oben angegeben Bedeutung haben,
   gegebenenfalls in Gegenwart eines Reaktionshilfsmittels und gegebenenfalls in Gegenwart eines Verdünnungsmittels umsetzt,
   oder wenn man
(b) Triazolinon-Derivate der allgemeinen Formel (IV) 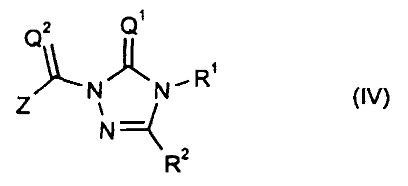 in welcher
   Q¹, Q², R¹ und R² die oben angegebene Bedeutung haben und
   - Z: für Halogen, Alkoxy, Aryloxy oder Arylalkoxy steht,
   mit Sulfonsäureamiden der allgemeinen Formel (V)

   R³-SO₂-NH₂ (V)

   in welcher
   - R³: die oben angegebene Bedeutung hat,
   gegebenenfalls in Gegenwart eines Säureakzeptors und gegebenenfalls in Gegenwart eines Verdünnungsmittels umsetzt,
   oder wenn man
(c) Triazolinone der allgemeinen Formel (II) 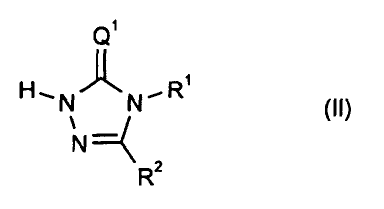 in welcher
   Q¹, R¹ und R² die oben angegebene Bedeutung haben,
   mit Sulfonsäureamid-Derivaten der allgemeinen Formel ((VI)

   R³-SO₂-NH-CQ²-Z (VI)

   in welcher
   Q² und R³ die oben angegebene Bedeutung haben und
   - Z: für für Halogen, Alkoxy, Aryloxy oder Arylalkoxy steht,
   gegebenenfalls in Gegenwart eines Säureakzeptors und gegebenenfalls in Gegenwart eines Verdünnungsmittels umsetzt,
   oder wenn man
(d) Triazolinone der allgemeinen Formel (II) 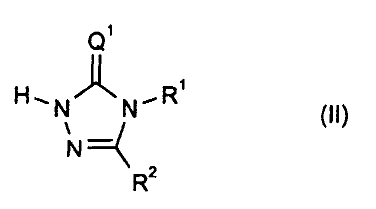 in welcher
   Q¹, R¹ und R² die oben angegebene Bedeutung haben,
   mit Sulfonsäurehalogeniden der allgemeinen Formel (VII)

   R³-SO₂-X (VII)

   in welcher
   - R³: die oben angegebene Bedeutung hat und
   - X: für Halogen steht,
   und Metallthiocyanaten der allgemeinen Formel (VIII)

   MQ²CN (VIII)

   in welcher
   - Q²: die oben angegebene Bedeutung hat und
   - M: für ein Alkalimetall oder ein Erdalkalimetalläqivalent steht,
   gegebenenfalls in Gegenwart eines Reaktionshilfsmittels und gegebenenfalls in Gegenwart eines Verdünnungsmittels umsetzt,
und gegebenenfalls die nach Verfahren (a), (b), (c) oder (d) erhaltenen Verbindungen der Formel (I) nach üblichen Methoden in Salze überführt.

Die neuen Sulfonylaminothiocarbonyl-triazolinone der allgemeinen Formel (I) zeichnen sich durch starke herbizide Wirksamkeit aus.

Gegenstand der Erfindung sind insbesondere Verbindungen der Formel (I), in welcher
- Q¹: für Sauerstoff steht,
- Q²: für Schwefel steht,
- R¹: für Wasserstoff, Hydroxy, Amino, für C₃-C₈-Alkylidenamino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht,
- R²: für Wasserstoff, Hydroxy, Mercapto, Amino, Fluor, Chlor, Brom, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Propenyloxy, Butenyloxy, Propinyloxy, Butinyloxy, Propenylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Trifluormethyl und/oder Methoxy substituiertes Phenyl, Benzyl, Phenoxy, Benzyloxy, Phenylthio, Benzylthio, Phenylamino oder Benzylamino steht, oder
- R¹ und R²: zusammen für gegebenenfalls verzweigtes Alkandiyl mit 3 bis 11 Kohlenstoffatomen stehen, und
- R³: für den Rest 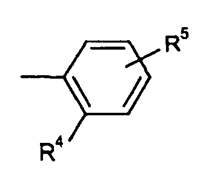 steht,
worin
R⁴ für Fluor, Chlor, Brom, für jeweils gegebenenfalls durch Fluor und/oder Chlor substituiertes Methyl, Ethyl, n- oder i-Propyl, für jeweils gegebenenfalls durch Fluor, Chlor, Methoxy oder Ethoxy substituiertes Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, Dimethylaminosulfonyl, Diethylaminosulfonyl, N-Methoxy-N-methylaminosulfonyl, Methoxyaminosulfonyl, Phenyl, Phenoxy, oder für jeweils gegebenenfalls durch Fluor, Chlor, Methoxy oder Ethoxy substituiertes Methoxycarbonyl, Ethoxycarbonyl, n- oder i-Propoxycarbonyl steht und
R⁵ für Wasserstoff, Methyl, Ethyl, Fluor, Chlor oder Brom steht; weiterhin
- R³: für den Rest 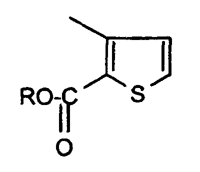 steht,
worin
R für Methyl, Ethyl, n- oder i-Propyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- bzw. Zwischenprodukte. Diese Restedefinitionen können untereinander, also auch zwischen den angegebenen bevorzugten Bereichen beliebig kombiniert werden.

Die bei den Restedefinitionen genannten Kohlenwasserstoffreste, wie Alkyl, Alkenyl oder Alkinyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Beispiele für die Bedeutung der Reste R¹, R² und R³ bei den erfindungsgemäßen Verbindungen der Formel (I) sind in der nachstehenden Gruppe aufgeführt. 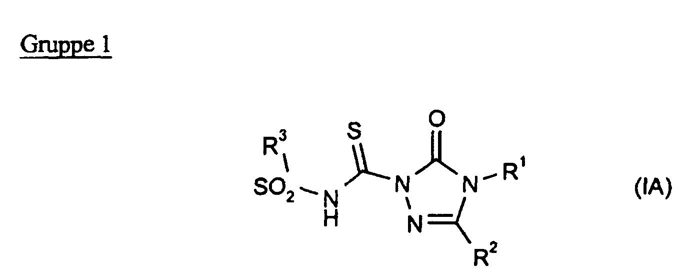
- R¹: hat hierbei beispielhaft die im Folgenden aufgeführten Bedeutungen:

Amino, n- oder i-Propylidenamino, Methyl, Ethyl, n- oder i-Propyl, n-, i-, s- oder t-Butyl, Dichlorpropyl, Dibrompropyl, Methoxypropyl, Propenyl, Butenyl, Propinyl, Butinyl, Methoxy, Ethoxy, n- oder i-Propoxy, Methylamino, Ethylamino, n- oder i-Propylamino, Propenyloxy, Dimethylamino, Cyclopropyl, Cyclobutyl, Cyclopentyl, Cyclohexyl, Methylcyclopropyl, Cyclopropylmethyl.

### R² hat hierbei beispielhaft die im Folgenden aufgeführten Bedeutungen:

Wasserstoff, Chlor, Brom, Methyl, Ethyl, n- oder i-Propyl, n-, i-, s- oder t-Butyl, Trifluormethyl, Methoxymethyl, Ethoxymethyl, Propenyl, Butenyl, Propinyl, Butinyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Fluormethylthio, Chlormethylthio, Fluorethylthio, Chlorethylthio, Difluorethylthio, Trifluorethylthio, Trifluorpropylthio, Methylamino, Ethylamino, n- oder i-Propylamino, n-, i-, s- oder t-Butylamino, Propenyloxy, Butenyloxy, Propinyloxy, Butinyloxy, Propenylthio, Butenylthio, Propenylamino, Butenylamino, Propinylamino, Butinylamino, Dimethylamino, Diethylamino, N-Methyl-ethylamino, N-Methyl-propylamino, N-Ethyl-propylamino, Cyclopropyl, Cyclopropyloxy, Cyclopropylamino, Cyclopropylmethyl, Cyclopropylmethoxy, Cyclopropylmethylthio, Phenyl, Phenoxy, Phenylthio, Phenylamino, Phenylmethyl, Phenylmethoxy, Phenylmethylthio, Phenyhnethylamino.

R¹ und R² haben hierbei auch beispielhaft zusammen die folgenden Bedeutungen:

Propan-1,3-diyl, Butan-1,4-diyl, Pentan-1,5-diyl, Hexan-1,6-diyl, Heptan-1,7-diyl, Undecan-1,11-diyl.

R³ hat hierbei beispielhaft die im Folgenden aufgeführten Bedeutungen:

2-Fluor-phenyl, 2-Chlor-phenyl, 2-Brom-phenyl, 2,6-Difluor-phenyl, 2,6-Dichlor-phenyl, 2-Methyl-phenyl, 2-Chlor-6-methyl-phenyl, 2-Trifluormethyl-phenyl, 2-(2-Fluor-ethyl)-phenyl, 2-(2-Chlor-ethyl)-phenyl, 2-(3-Fluor-propyl)-phenyl, 2-(3-Chlor-propyl)-phenyl, 2-(3,3,3-Trifluor-propyl)-phenyl, 2-(3,3,3-Trifluor-1-propenyl)-phenyl, 2-Methoxyphenyl, 2-Ethoxy-phenyl, 2-Difluormethoxy-phenyl, 2-Trifluormethoxy-phenyl, 2-(2-Fluor-ethoxy)-phenyl, 2-(2-Chlor-ethoxy)-phenyl, 2-(2-Methoxy-ethoxy)-phenyl, 2-Methylthio-phenyl, 2-Ethylthio-phenyl, 2-Methylsulfinyl-phenyl, 2-Ethylsulfinyl-phenyl, 2-Methylsulfonyl-phenyl, 2-Ethylsulfonyl-phenyl, 2-Dimethylaminosulfonyl-phenyl, 2-Phenyl-phenyl, 2-Methoxycarbonyl-phenyl, 2-Ethoxycarbonyl-phenyl, 2-Methoxycarbonyl-3-thienyl, 2-Ethoxycarbonyl-3-thienyl.

Verwendet man beispielsweise 2,6-Difluor-phenylisocyanat und 5-Ethyl-4-methoxy-2,4-dihydro-3H-1,2,4-triazol-3-thion als Ausgangsstoffe, so kann der Reaktionsablauf beim erfindungsgemäßen Verfahren (a) durch das folgende Formelschema skizziert werden:

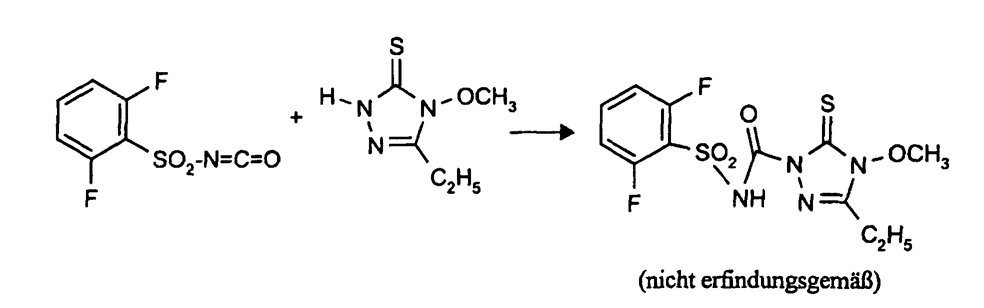

Verwendet man beispielsweise 2-Methylthio-benzolsulfonamid und 2-Chlorcarbonyl-4-dimethylamino-5-methyl-2,4-dihydro-3H-1,2,4-triazol-3-thion als Ausgangsstoffe, so kann der Reaktionsablauf beim erfindungsgemäßen Verfahren (b) durch das folgende Formelschema skizziert werden:

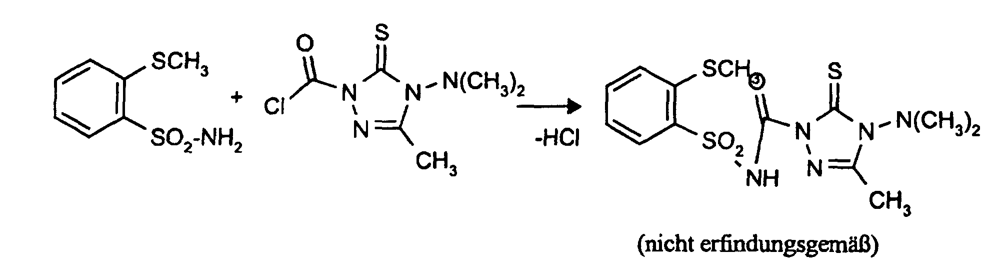

Verwendet man beispielsweise N-Methoxythiocarbonyl-2-methoxy-benzolsulfonamid und 5-Ethoxy-4-methyl-2,4-dihydro-3H-1,2,4-triazol-3-on als Ausgangsstoffe, so kann der Reaktionsablauf beim erfindungsgemäßen Verfahren (c) durch das folgende Formelschema skizziert werden:

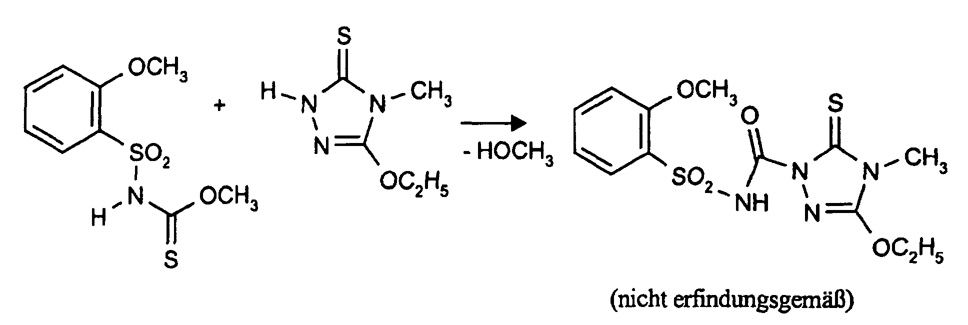

Verwendet man beispielsweise 4-Ethyl-5-ethylthio-2,4-dihydro-3H-1,2,4-triazol-3-thion und 2-Brom-benzolsulfonsäurechlorid sowie Kalium-thiocyanat als Ausgangsstoffe, so kann der Reaktionsablauf nach dem erfindungsgemäßen Verfahren (d) durch das folgende Reaktionsschema skizziert werden:

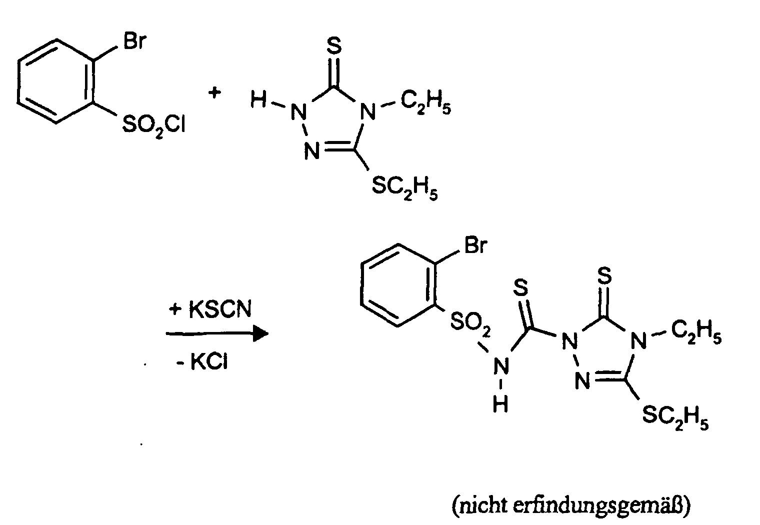

Die bei den erfindungsgemäßen Verfahren (a), (c) und (d) zur Herstellung von Verbindungen der Formel (I) als Ausgangsstoffe zu verwendenden Triazolinone sind durch die Formel (II) allgemein definiert.

In der Formel (II) haben Q¹, R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q¹, R¹ und R² angegeben wurden.

Die Triazolinone der allgemeinen Formel (II) sind bekannt und/oder können nach an sich bekannten Verfahren hergestellt werden (vgl. Arch. Pharm. 301 (1968), 827; loc. cit. 307 (1974), 889; Bull. Soc. Chim. France 1962, 1365; loc. cit. 1975, 1191;Chem. Ber. 90 (1957), 909-921; loc. cit. 98 (1965), 3025-3099; loc. cit. 102 (1969), 755; J. Heterocycl. Chem. 15 (1978), 237-240; J. Indian Chem. Soc. 6 (1929), 565; Liebigs Ann. Chem. 637 (1960), 135; Monatshefte Chemie 123 (1992), 257; Tetrahedron 32 (1976), 2347-2352; Helv. Chim. Acta 63 (1980), 841-859; J. Chem. Soc. C 1967, 746-751; loc. cit. 1970, 26-34; J. Chem. Soc. Perkin I 1973, 2644; Fen Fak. Derg., Seri A (Ege Univ.) 7 (1984), 1-6 - zitiert in Chem. Abstracts 101:90846m; EP-A 283876; EP-A 294666; EP-A 298371; EP-A 301946; EP-A 305844; EP-A 341489; EP-A 362633; EP-A 370293; EP-A 391187; EP-A 398096; EP-A 398097; EP-A 399294; EP-A 415196; EP-A 422469; EP-A 425948; EP-A 431291; EP-A 477646; EP-A 502307; EP-A 503437; EP-A 505819; EP-A 511569; EP-A 513621; DE-A 2336827; DE-A 3839206; DE-A 3916208; DE-A 3916930; DD-P 64970; WO-A 93/04050; Herstellungsbeispiele).

Die beim erfindungsgemäßen Verfahren (a) zur Herstellung von Verbindungen der Formel (I) weiter als Ausgangsstoffe zu verwendenden Sulfonylisothiocyanate sind durch die Formel (III) allgemein definiert.

In Formel (III) haben Q² und R³ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Q² und R³ angegeben wurde.

Die Ausgangsstoffe der Formel (III) sind bekannt und/oder können nach an sich bekannten Verfahren hergestellt werden (vgl. US-P 4127405, US-P 4169719, US-P 4371391, EP-A 7687, EP-A 13480, EP-A 21641, EP-A 23141, EP-A 23422, EP-A 30139, EP-A 35893, EP-A 44808, EP-A 44809, EP-A 48143, EP-A 51466, EP-A 64322, EP-A 70041, EP-A 173312).

Die beim erfindungsgemäßen Verfahren (b) zur Herstellung der Verbindungen der allgemeinen Formel (I) als Ausgangsstoffe zu verwendenden Triazolinon-Derivate sind durch die Formel (IV) allgemein definiert. In der Formel (IV) haben Q¹, Q², R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q¹, Q², R¹ und R² angegeben wurden; Z steht vorzugsweise für Fluor, Chlor, Brom, Methoxy, Ethoxy, Benzyloxy, Phenoxy, Halogen- oder Nitrophenoxy, insbesondere für Methoxy, Phenoxy oder 4-Nitro-phenoxy.

Die Ausgangsstoffe der Formel (IV) sind teilweise aus der Literatur bekannt (vgl. EP-A 294 666, EP-A 625 515 und EP-A 415 196).

Triazolinon-Derivate der allgemeinen Formel (IV), bei denen Q¹, Q², R² und Z wie oben angegeben definiert sind und bei denen R¹ für Wasserstoff, Hydroxy, für gegebenenfalls durch Fluor, Chlor, Brom, Cyano, C₁-C₄-Alkoxy, C₁-C₄-alkyl-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für gegebenenfalls durch Fluor, Chlor, Cyano, C₁-C₄-Alkoxy oder C₁-C₄-alkoxy-carbonyl substituiertes C₁-C₆-Alkoxy, für C₃-C₆-Alkenyloxy, für jeweils gegebenenfalls durch Fluor, Chlor, Brom, Cyano und/oder C₁-C₄-Alkyl substituiertes C₃-C₆-Cycloalkyl oder C₃-C₆-Cycloalkyl-C₁-C₄-alkyl steht, sind als neue Stoffe auch Gegenstand der vorliegenden Erfindung.

Man erhält die neuen Verbindungen der allgemeinen Formel (IV), wenn man Triazolinone der allgemeinen Formel (II) 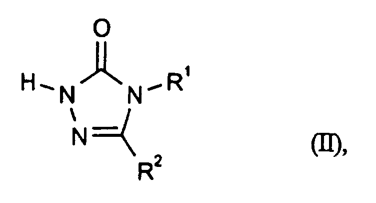 in welcher
R¹ und R² die oben angegebene Bedeutung haben,
mit Thiokohlensäurederivaten der allgemeinen Formel (IX)

Z-CQ²-Z¹ (IX)

in welcher
Z und Q² die oben angegebene Bedeutung haben und
- Z¹: für Halogen, Alkoxy, Aralkoxy oder Aryloxy steht,
gegebenenfalls in Gegenwart eines Säureakzeptors, wie z.B. Natrium- oder Kaliumhydroxid, Natrium- oder Kalium-t-butylat, und gegebenenfalls in Gegenwart eines Verdünnungsmittels, wie z.B. Methylenchlorid, Tetrahydrofuran oder Dimethoxyethan und/oder Wasser, bei Temperaturen zwischen 0°C und 100°C umsetzt.

Die beim erfindungsgemäßen Verfahren (b) zur Herstellung der Verbindungen der allgemeinen Formel (I) weiter als Ausgangsstoffe zu verwendenden Sulfonsäureamide sind durch die Formel (V) allgemein definiert. In der Formel (V) hat R³ vorzugsweise bzw. insbesondere diejenige Bedeutung, die bereits oben im Zusammenhang mit der Beschreibung der Verbindungen der Formel (I) als bevorzugt bzw. als insbesondere bevorzugt für R³ angegeben wurde.

Die Ausgangsstoffe der Formel (V) sind bekannt und/oder können nach an sich bekannten Verfahren hergestellt werden (vgl. US-P 4127405, US-P 4169719, US-P 4371391, EP-A 7687, EP-A 13480, EP-A 21641, EP-A 23141, EP-A 23422, EP-A 30139, EP-A 35893, EP-A 44808, EP-A 44809, EP-A 48143, EP-A 51466, EP-A 64322, EP-A 70041, EP-A 173312).

Die beim erfindungsgemäßen Verfahren (c) zur Herstellung der Verbindungen der Formel (I) als Ausgangsstoffe zu verwendenden Sulfonsäureamid-Derivate sind durch die Formel (VI) allgemein definiert. In der Formel (VI) haben Q² und R³ vorzugsweise bzw. insbesondere diejenige Bedeutung, die bereits oben im Zusammenhang mit der Beschreibung der Verbindungen der Formel (I) als bevorzugt bzw. als insbesondere bevorzugt für Q² und R³ angegeben wurde; Z steht vorzugsweise für Fluor, Chlor, Brom, Methoxy, Ethoxy, Benzyloxy oder Phenoxy, insbesondere für Methoxy oder Phenoxy.

Die Ausgangsstoffe der Formel (VI) sind bekannt und/oder können nach an sich bekannten Verfahren hergestellt werden.

Die beim erfindungsgemäßen Verfahren (d) zur Herstellung der Verbindungen der Formel (I) als Ausgangsstoffe zu verwendenden Sulfonsäurehalogenide sind durch die Formel (VII) allgemein definiert. In der Formel (VII) hat R³ vorzugsweise bzw. insbesondere diejenige Bedeutung, die bereits oben im Zusammenhang mit der Beschreibung der Verbindungen der Formel (I) als bevorzugt bzw. als insbesondere bevorzugt für R³ angegeben wurde; X steht vorzugsweise für Fluor, Chlor oder Brom, insbesondere für Chlor.

Die Ausgangsstoffe der Formel (VII) sind bekannt und/oder können nach an sich bekannten Verfahren hergestellt werden.

Die erfindungsgemäßen Verfahren (a), (b), (c) und (d) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol; Ether wie Diethyl- und Dibutylether, Glykoldimethylether und Diglykoldimethylether, Tetrahydrofuran und Dioxan; Ketone wie Aceton, Methyl-ethyl-, Methyl-isopropyl- und Methyl-isobutyl-keton; Ester wie Essigsäuremethylester und -ethylester; Nitrile wie z.B. Acetonitril und Propionitril; Amide wie z.B. Dimethylformamid, Dimethylacetamid und N-Methylpyrrolidon, sowie Dimethylsulfoxid, Tetramethylensulfon und Hexamethylphosphorsäuretriamid.

Als Reaktionshilfsmittel bzw. als Säureakzeptoren können bei den erfindungsgemäßen Verfahren (a), (b), (c) und (d) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo[5,4,0]-undec-7-en (DBU) und 1,4-Diazabicyclo-[2,2,2]-octan (DABCO).

Die Reaktionstemperaturen können bei den erfindungsgemäßen Verfahren (a), (b), (c) und (d) in einem größeren Bereich variiert werden. Im allgemeinen arbeitet man bei Temperaturen zwischen -20°C und +150°C, vorzugsweise bei Temperaturen zwischen 0°C und +100°C.

Die erfindungsgemäßen Verfahren (a), (b), (c) und (d) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b), (c) und (d) werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c) und (d) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria. Matricaria, Anthemis, Galinsoga, Chenopodium, Urtica, Senecio, Amaranthus. Portulaca, Xanthium, Convolvulus, Ipomoea, Polygonum, Sesbania, Ambrosia. Cirsium, Carduus, Sonchus, Solanum, Rorippa, Rotala, Lindernia, Lamium. Veronica, Abutilon, Emex, Datura, Viola, Galeopsis, Papaver, Centaurea. Trifolium, Ranunculus, Taraxacum.

Dikotyle Kulturen der Gattungen: Gossypium, Glycine, Beta, Daucus, Phaseolus. Pisum, Solanum, Linum, Ipomoea, Vicia, Nicotiana, Lycopersicon, Arachis. Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus. Sorghum, Agropyron, Cynodon, Monochoria, Fimbristylis, Sagittaria, Eleocharis, Scirpus, Paspalum, Ischaemum, Sphenoclea, Dactyloctenium, Agrostis. Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im NachauflaufVerfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolin-säure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 10 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

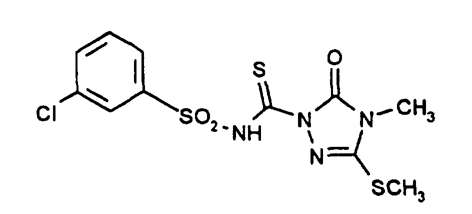

(nach Verfahren (a))
Eine Mischung aus 3,0 g (20,7 mMol) 4-Methyl-5-methylthio-2,4-dihydro-3H-1,2,4-triazol-3-on, 4,8 g (20,7 mMol) 3-Chlor-phenylsulfonylisothiocyanat, 0,3 g Triethylamin und 60 ml Acetonitril wird 12 Stunden unter Rückfluß erhitzt und anschließend im Wasserstrahlvakuum eingeengt. Der Rückstand wird mit Diethylether verrührt und das kristallin angefallene Produkt durch Absaugen isoliert.

Man erhält 2,1 g (27% der Theorie) 2-(3-Chlor-phenylsulfonyl-aminothiocarbonyl)-4-methyl-5-methylthio-2,4-dihydro-3H-1,2,4-triazol-3-on vom Schmelzpunkt 141°C.

### Beispiel 2

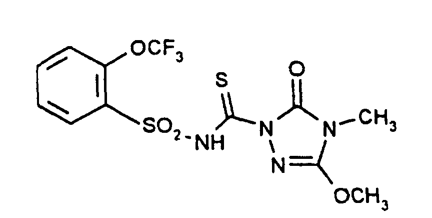

(nach Verfahren (b))

Eine Mischung aus 3,2 g (12 mMol) 5-Methoxy-4-methyl-2-phenoxythiocarbonyl-2,4-dihydro-3H-1,2,4-triazol-3-on, 2,9 g (12 mMol) 2-Trifluormethoxy-benzolsulfonamid, 1,9 g (12,5 mMol) 1,8-Diazabicyclo-[5,4,0]-undec-7-en (DBU) und 30 ml Dioxan wird 12 Stunden bei 20°C gerührt. Anschließend wird mit Methylenchlorid und Wasser verdünnt und mit 2N-Salzsäure auf pH=3 eingestellt. Dann wird die organische Phase abgetrennt, mit Natriumsulfat getrocknet und filtriert.

Das Filtrat wird im Wasserstrahlvakuum eingeengt, der Rückstand durch Digerieren mit Diethylether zur Kristallisation gebracht und das Produkt durch Absaugen isoliert.

Man erhält 3,1 g (63% der Theorie) 5-Methoxy-4-methyl-2-(2-trifluormethoxyphenylsulfonyl-aminothiocarbonyl)-2,4-dihydro-3H-1,2,4-triazol-3-on vom Schmelzpunkt 139°C.

### Beispiel 3

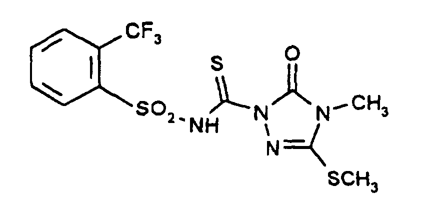

(nach Verfahren (b))

Zu einer Lösung von 28,1 g (0,10 Mol) 4-Methyl-5-methylthio-2-phenoxythiocarbonyl-2,4-dihydro-3H-1,2,4-triazol-3-on in 300 ml Acetonitril werden bei 20°C nacheinander 28,1 g (0,125 Mol) 2-Trifluormethyl-benzolsulfonamid und eine Lösung von 19 g 1,8-Diazabicyclo-[5,4,0]-undec-7-en (DBU) in 50 ml Acetonitril gegeben Die Reaktionsmischung wird bei 20°C gerührt, bis eine klare Lösung entstanden ist. Dann wird im Wasserstrahlvakuum eingeengt, der Rückstand mit Wasser und Methylenchlorid (jeweils 300 ml) versetzt und mit 2N-Salzsäure angesäuert. Die organische Phase wird abgetrennt, die wässrige Phase mit 100 ml Methylenchlorid nachextrahiert, die vereinigten organischen Phasen mit Magnesiumsulfat getrocknet und filtriert. Das Filtrat wird im Wasserstrahlvakuum eingeengt und der Rückstand aus Isopropanol kristallisiert.

Man erhält 29,6 g (72% der Theorie) 4-Methyl-5-methylthio-2-(2-trifluormethylphenylsulfonyl-aminothiocarbonyl)-2,4-dihydro-3H-1,2,4-triazol-3-on vom Schmelzpunkt 154°C.

Analog zu den Beispielen 1, 2 und 3 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

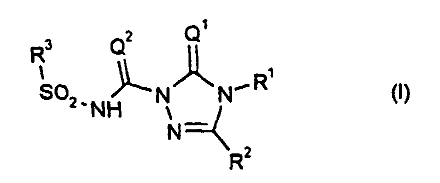

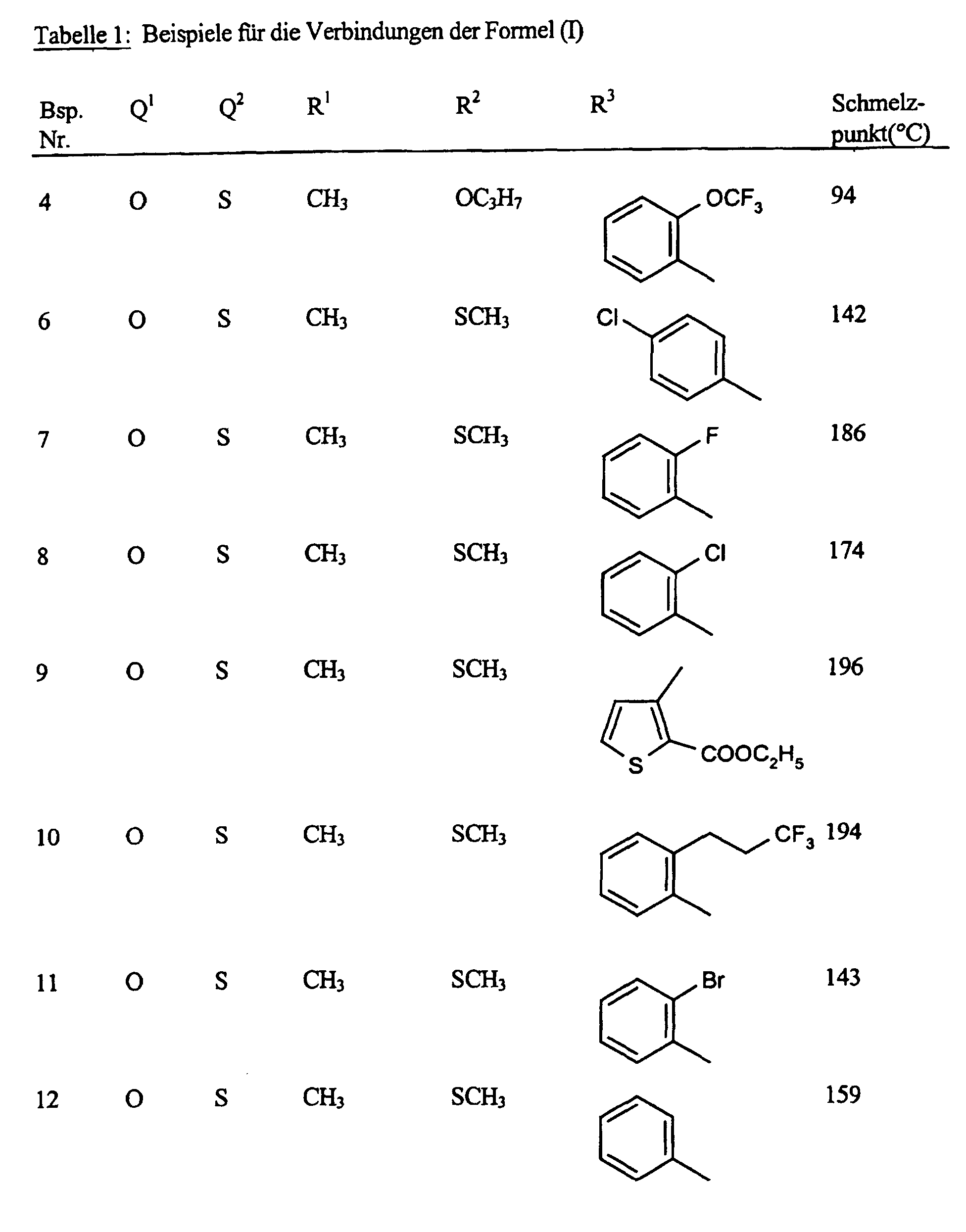

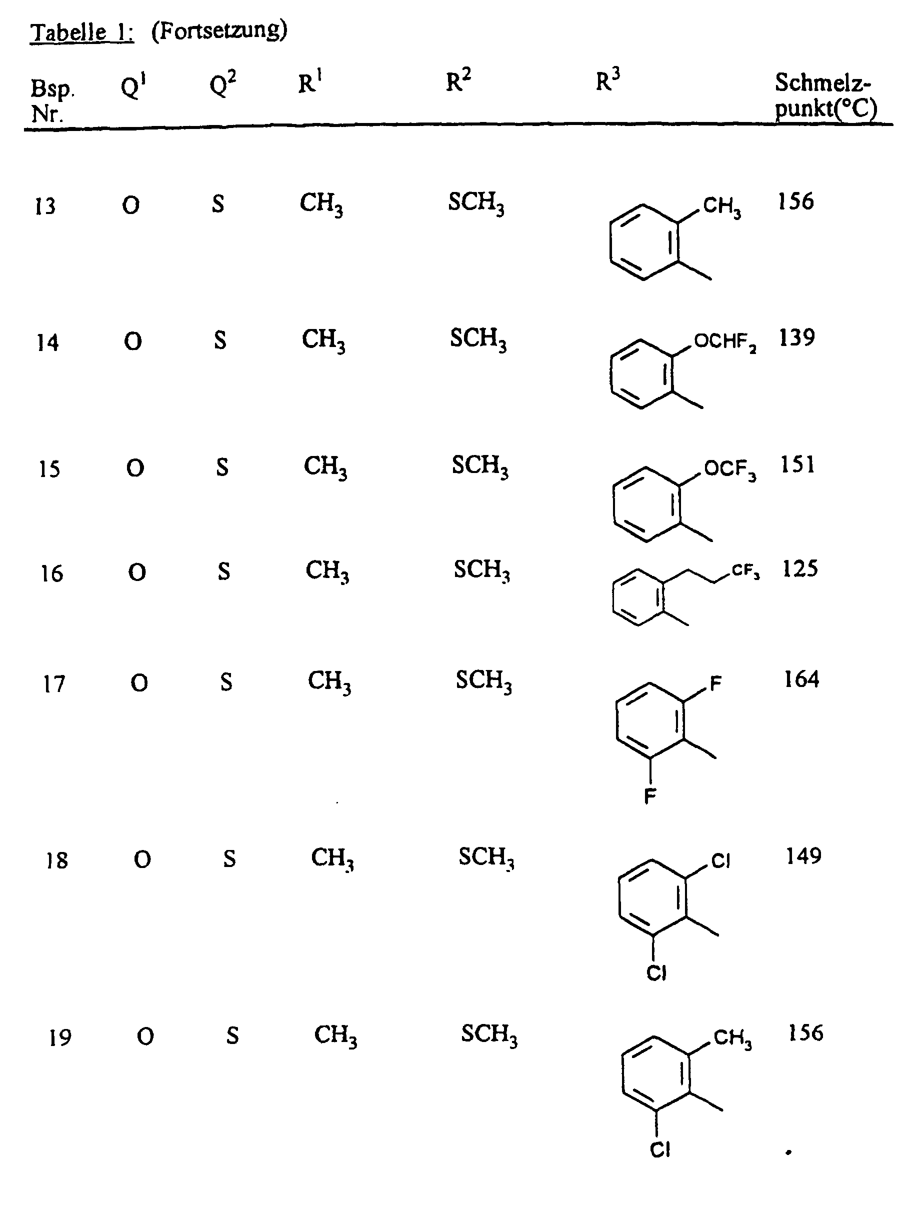

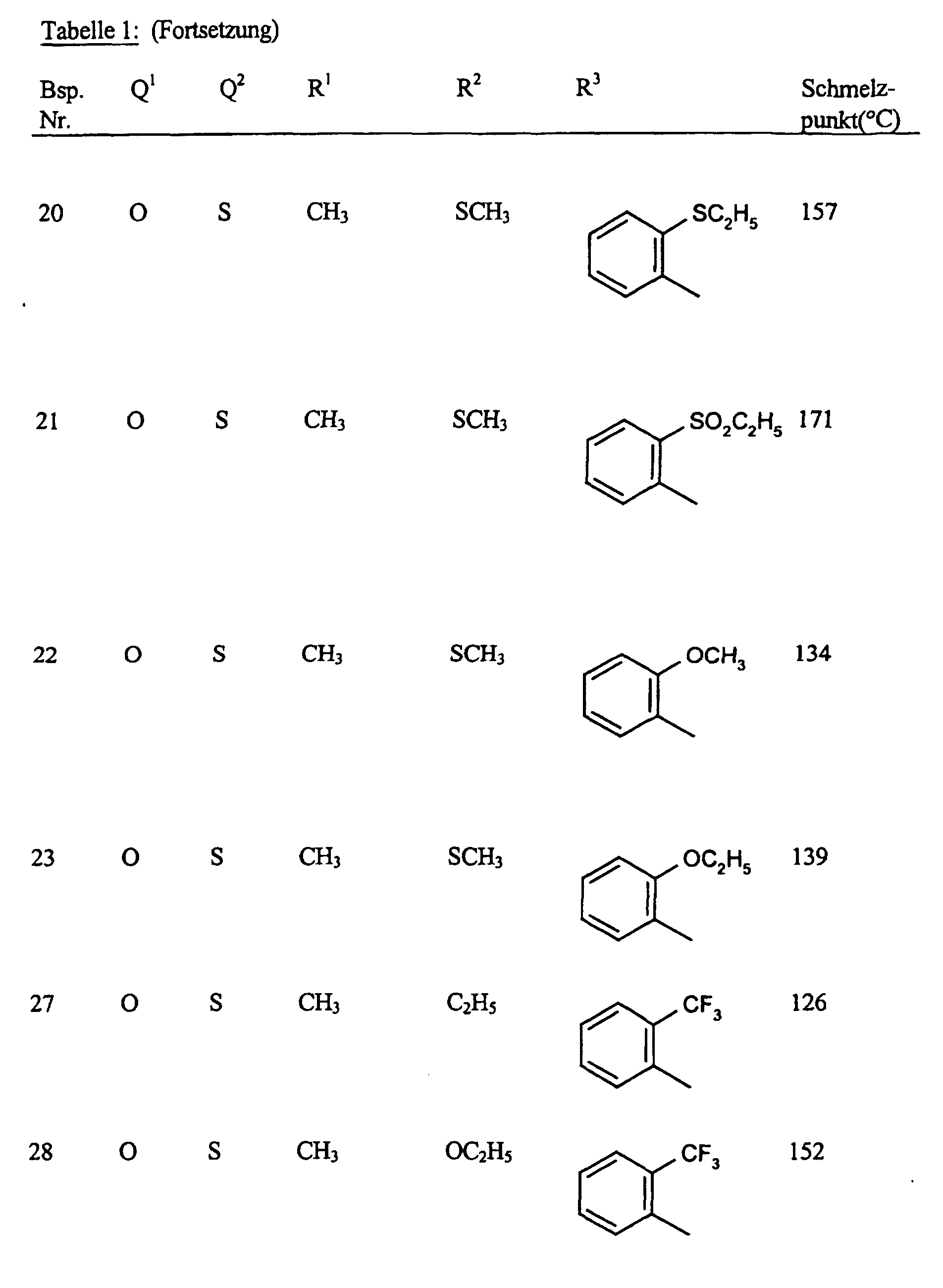

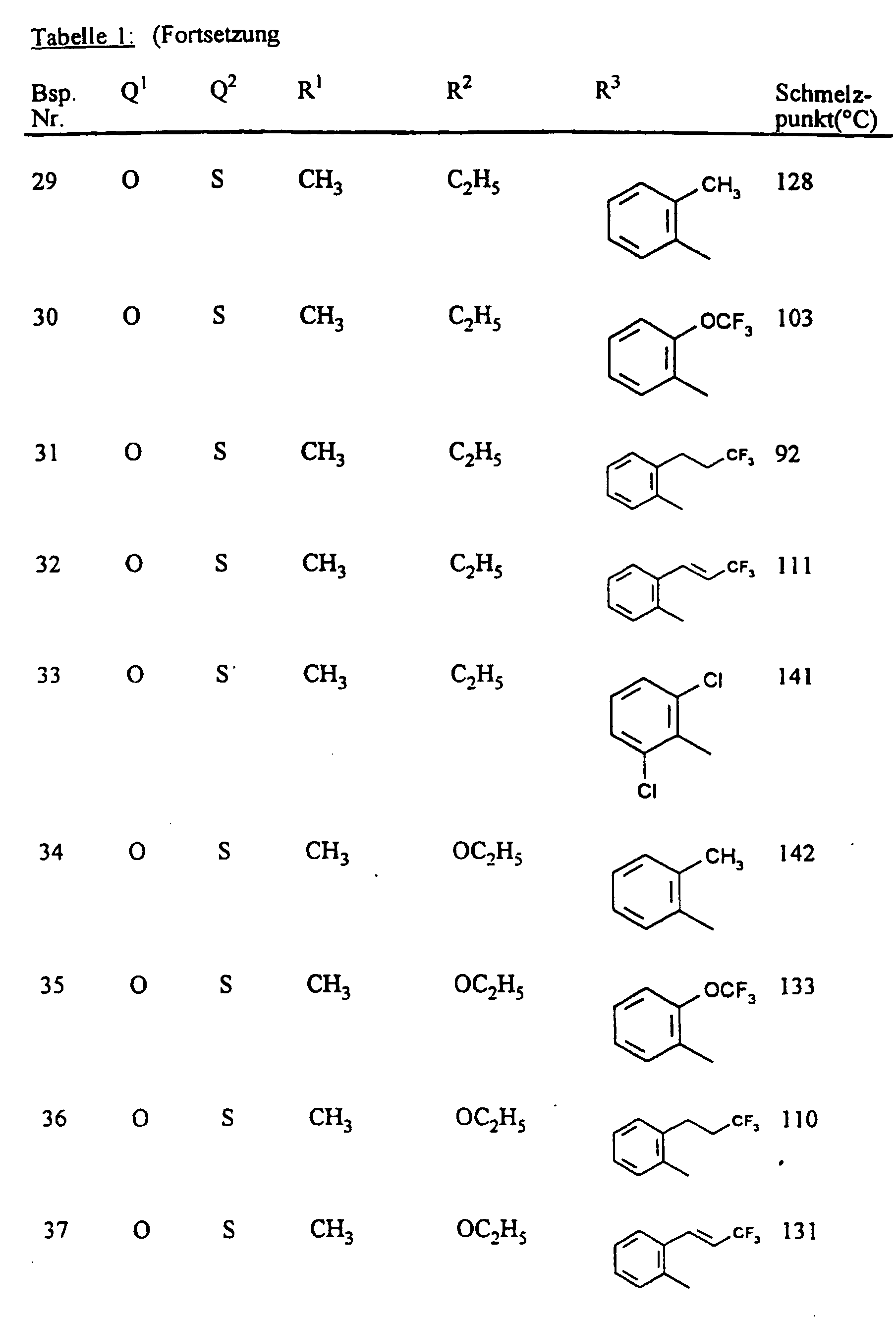

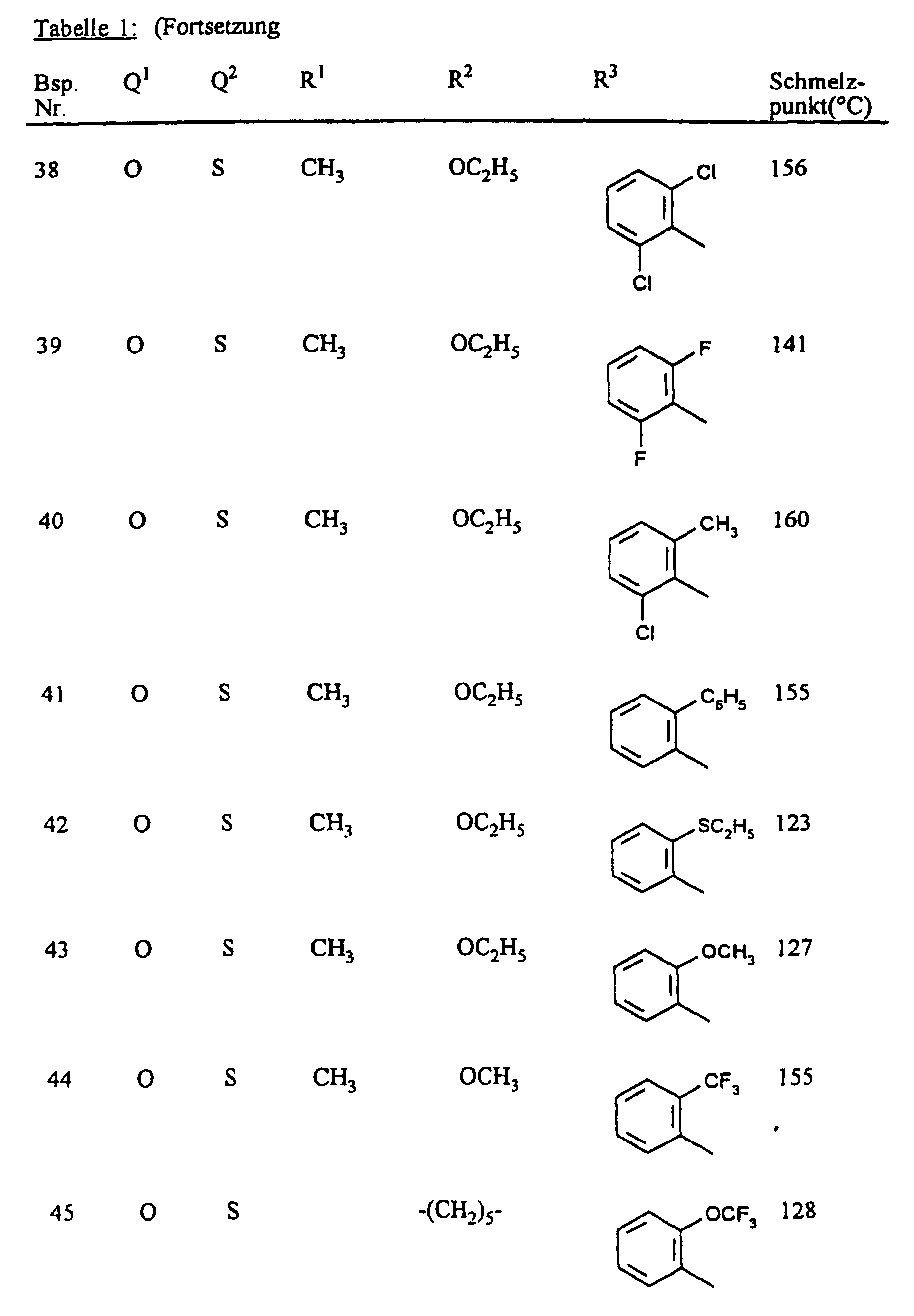

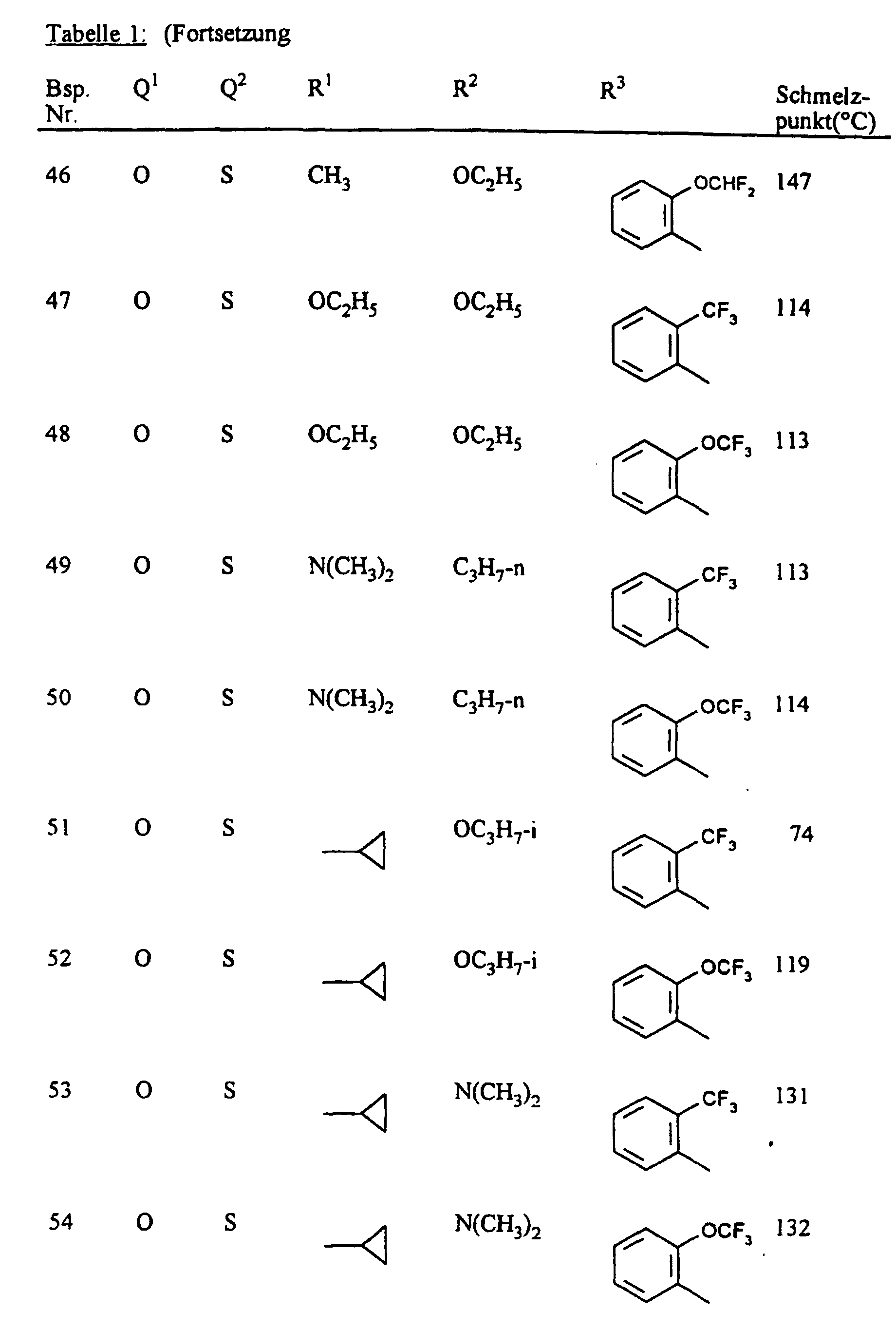

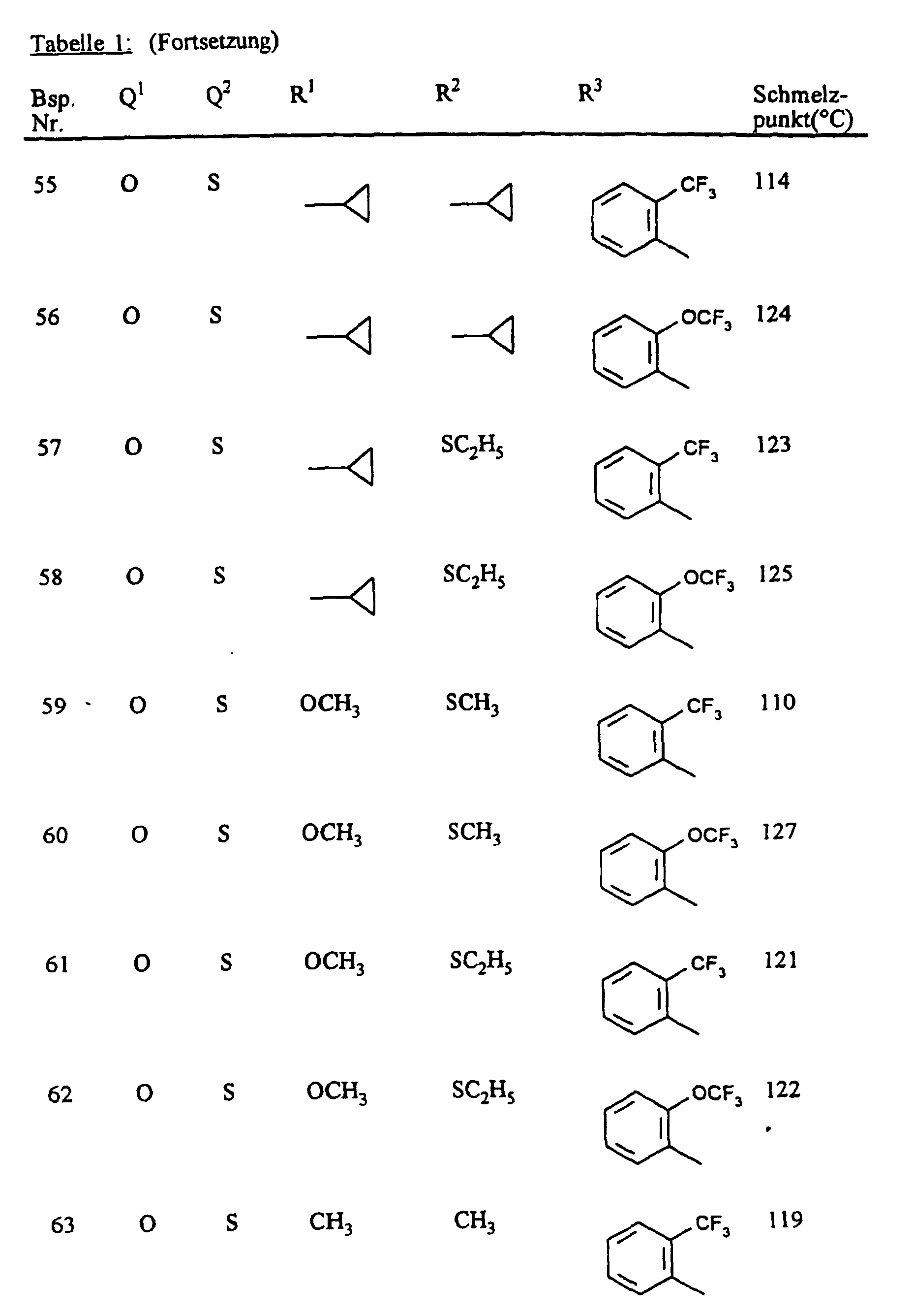

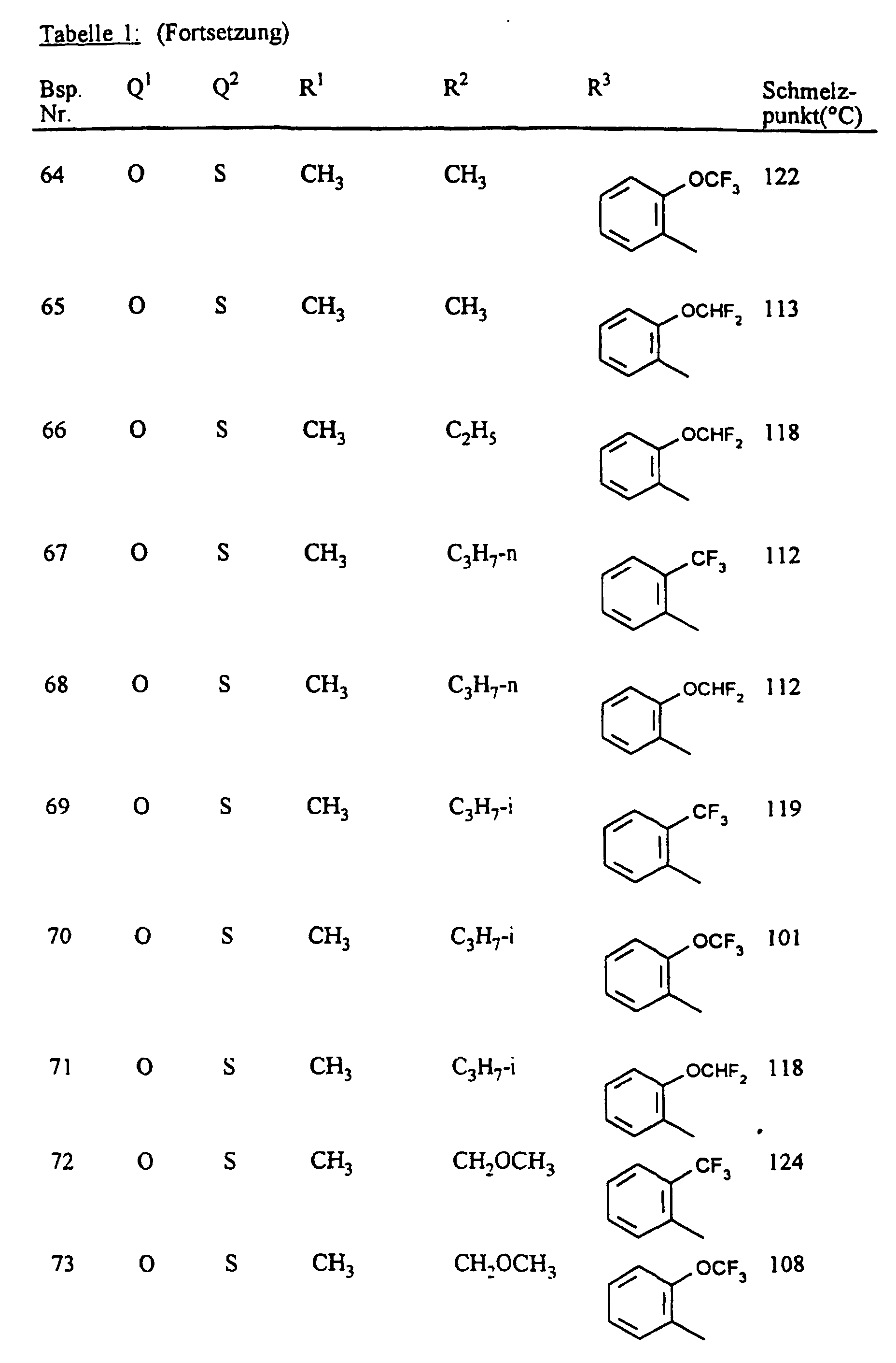

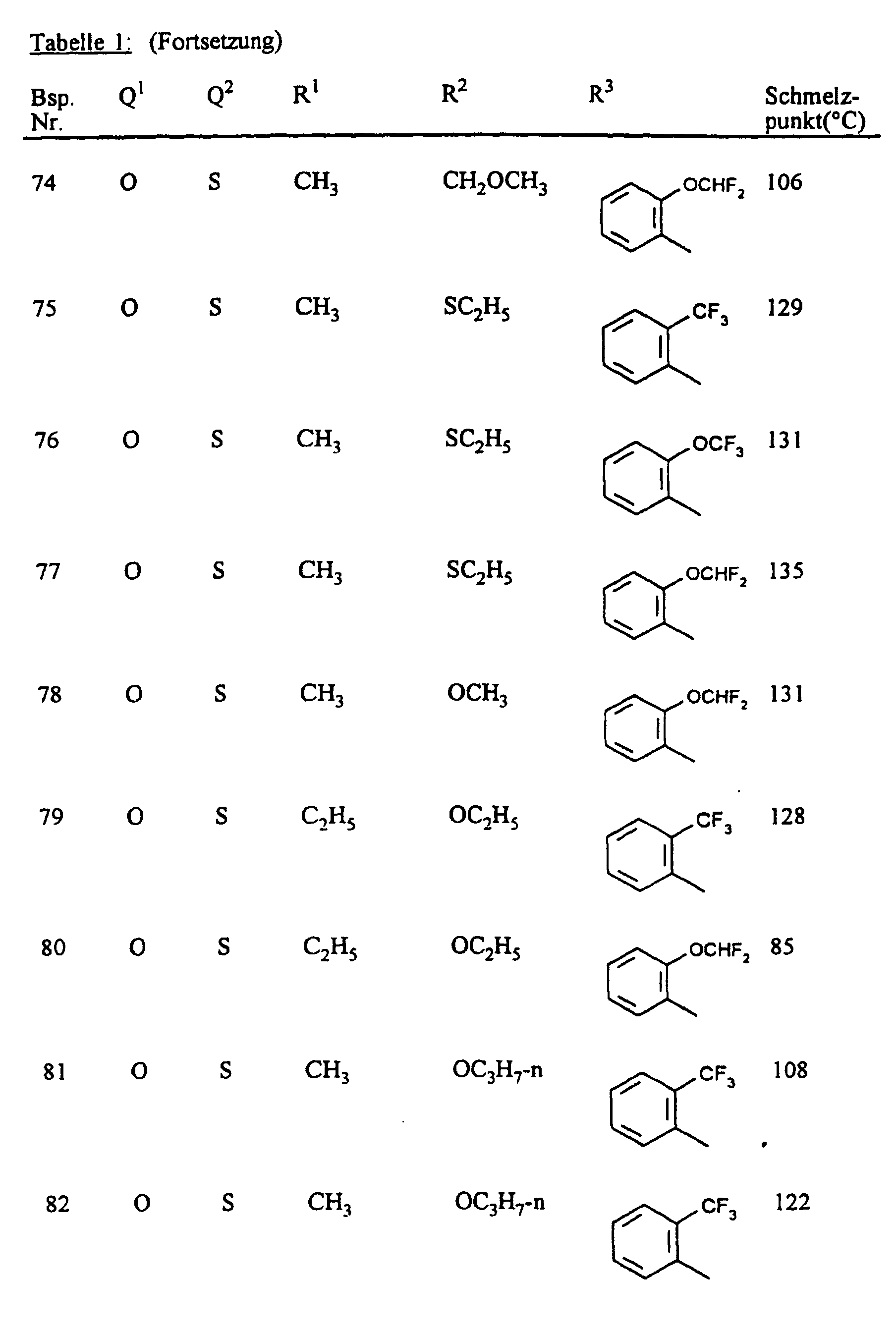

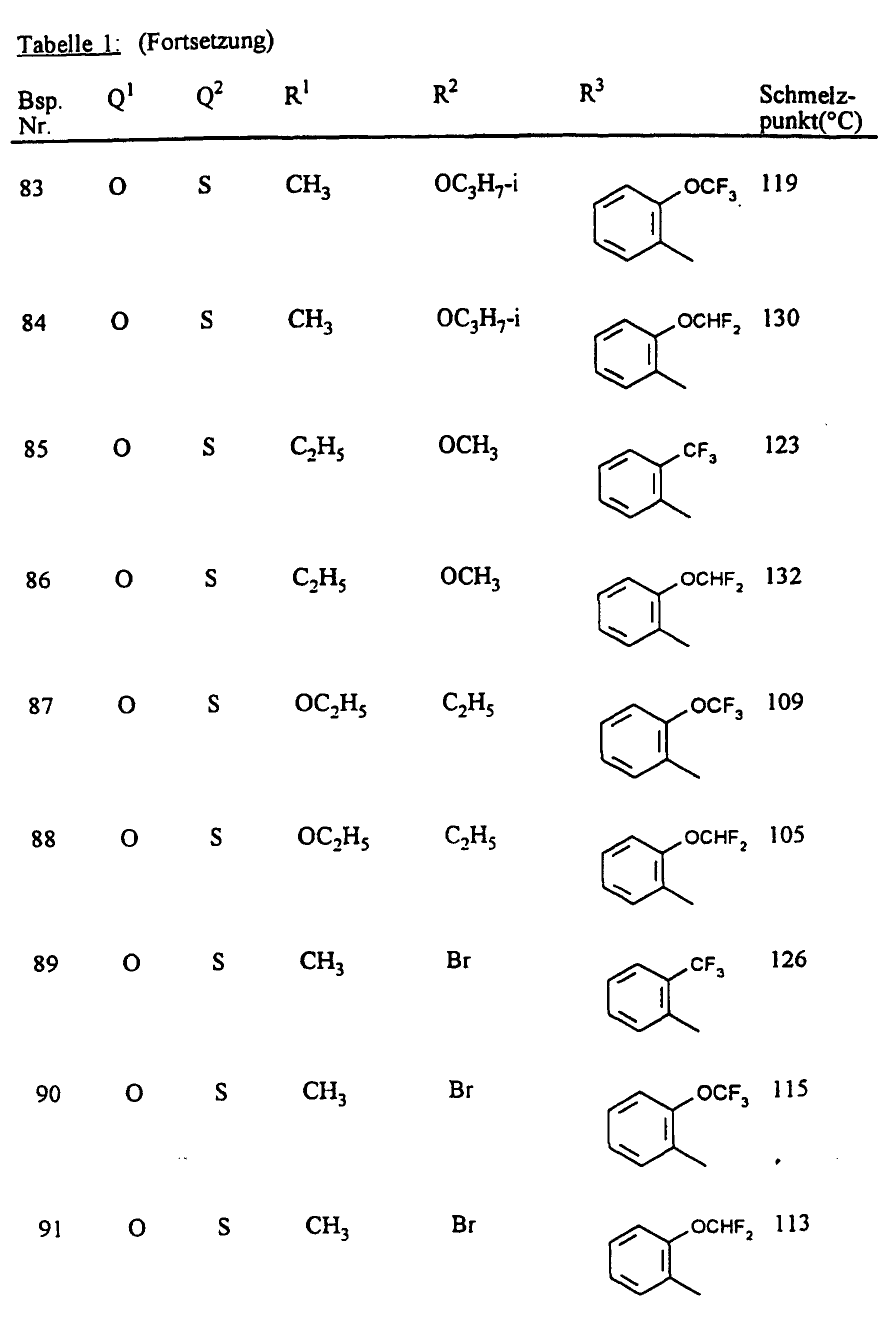

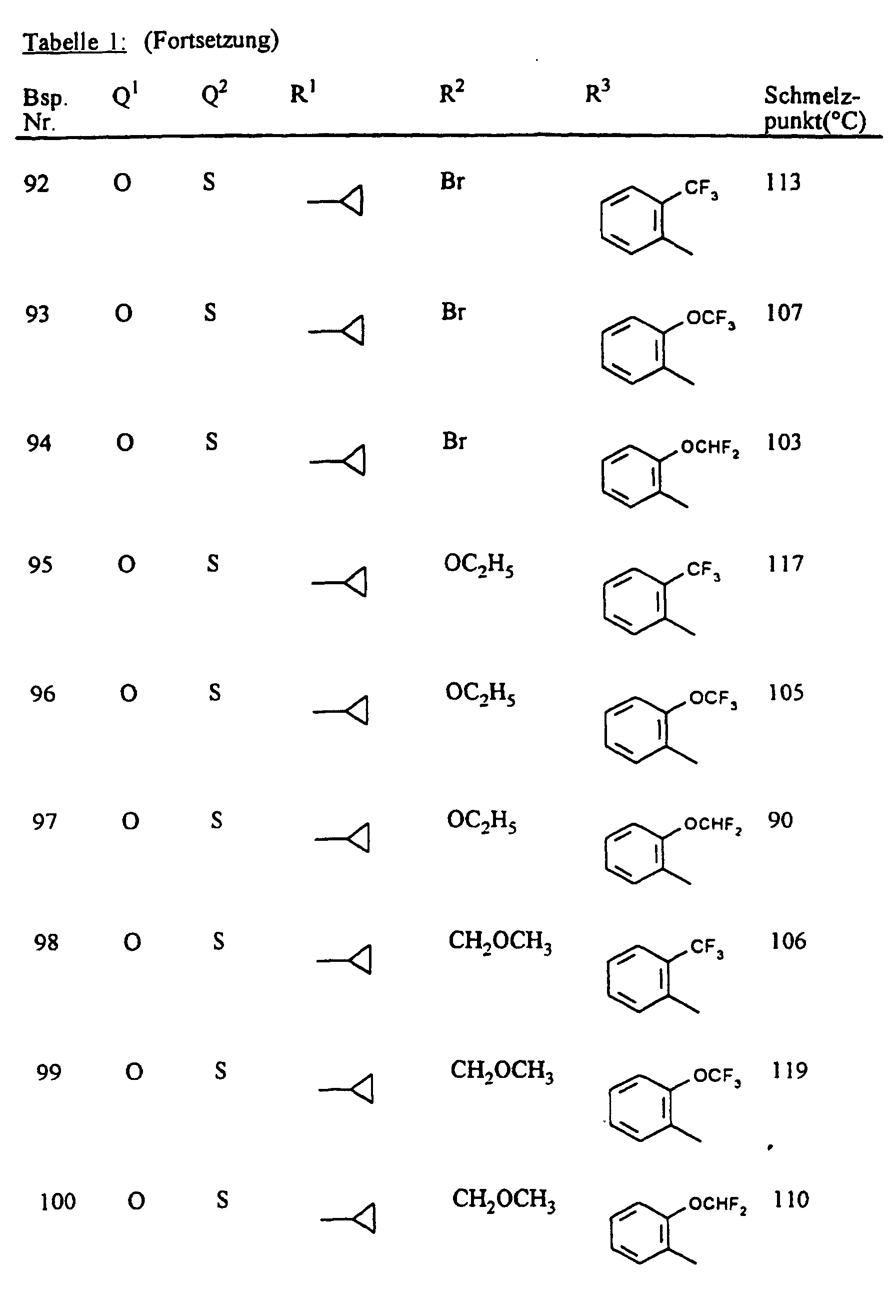

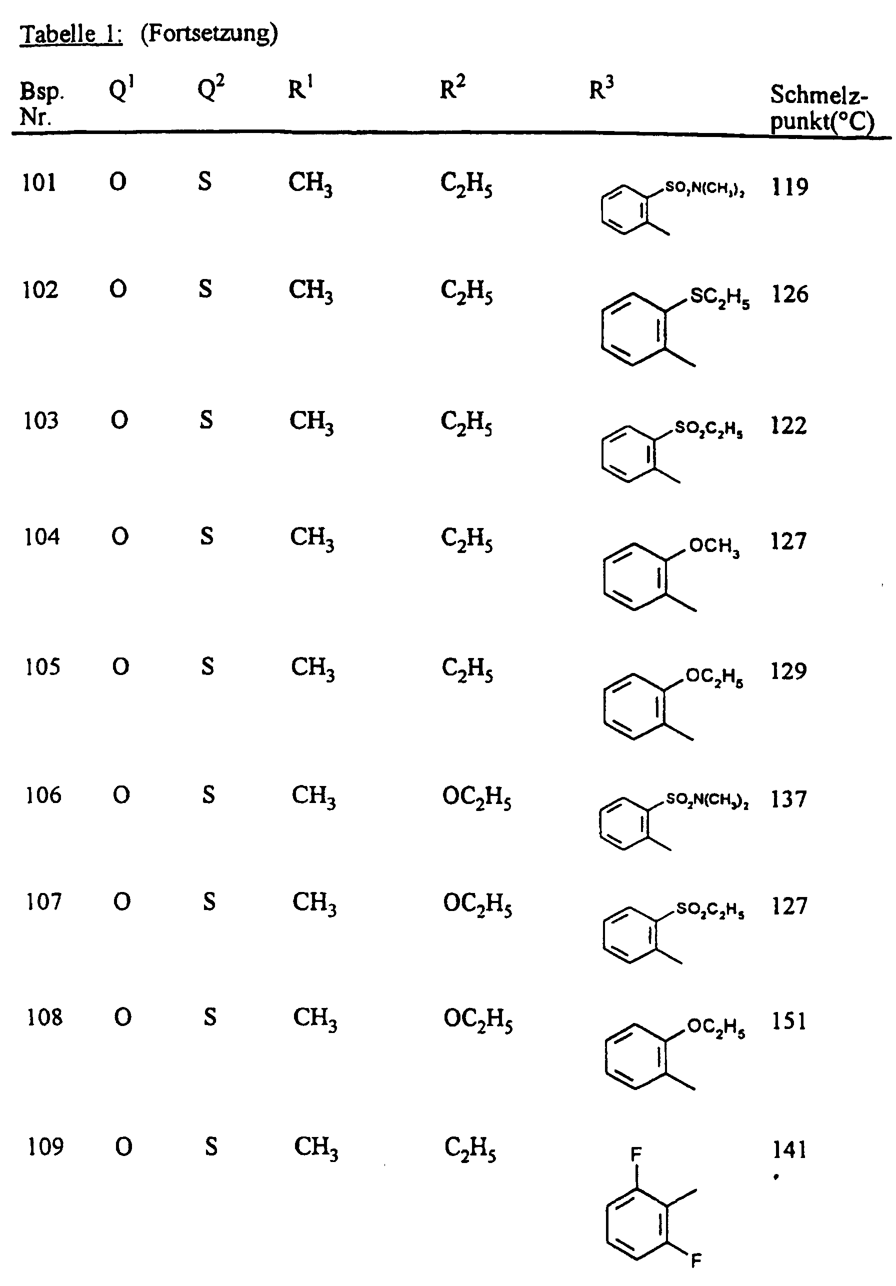

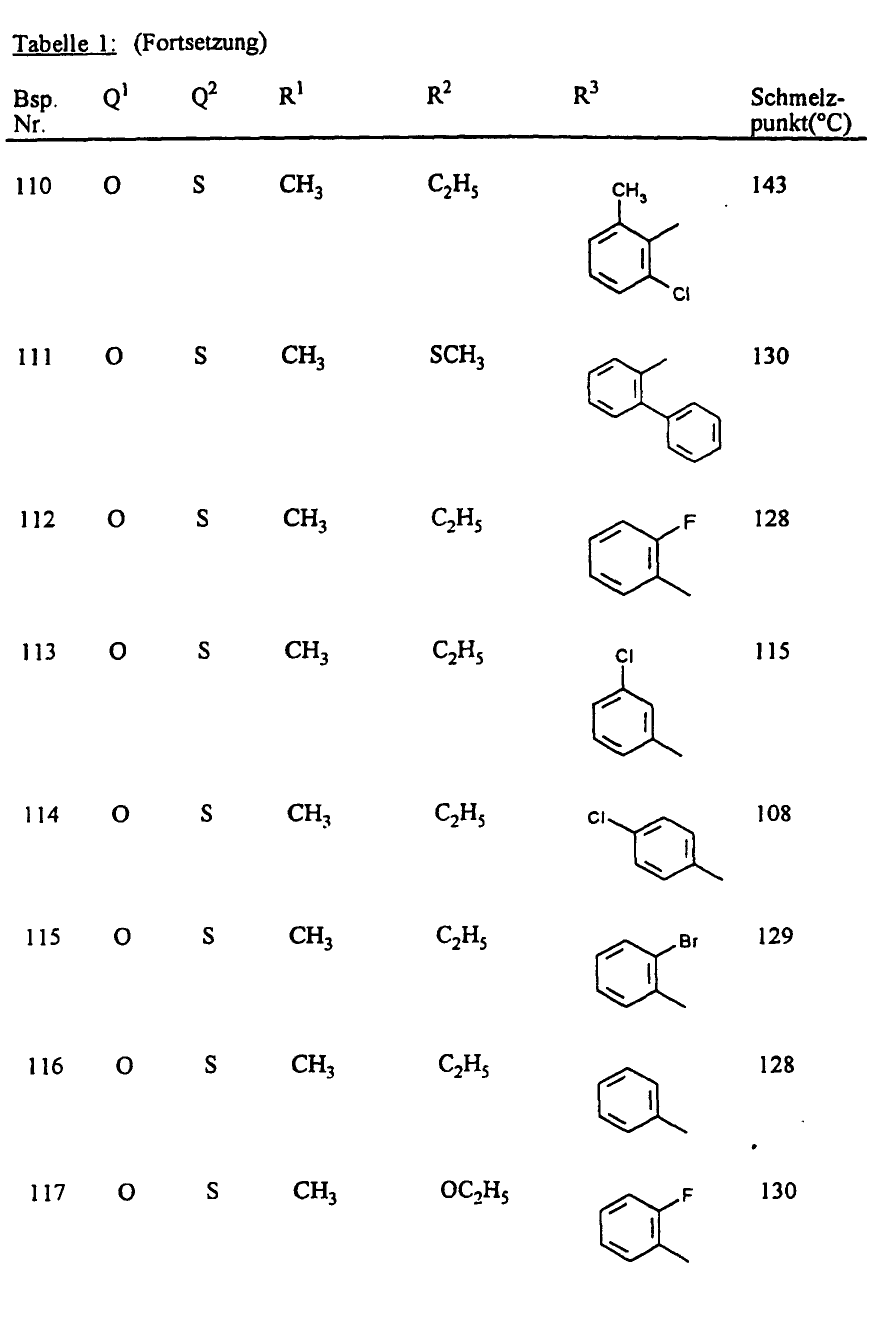

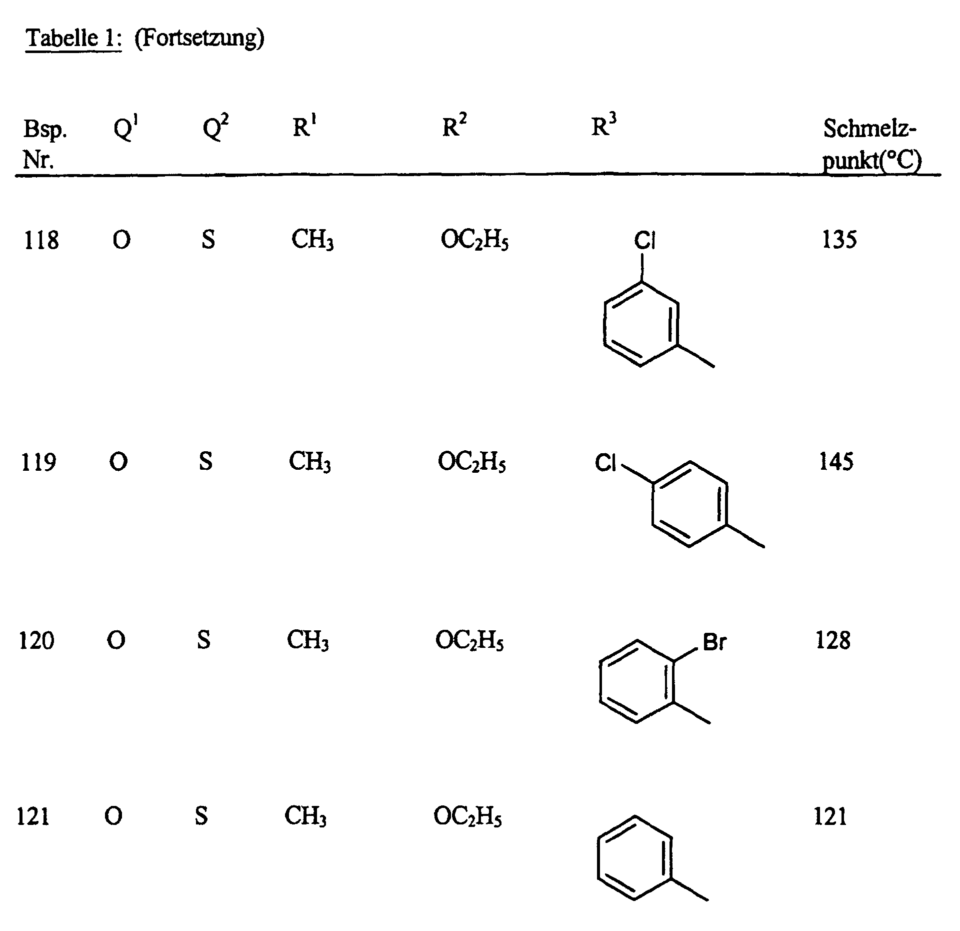

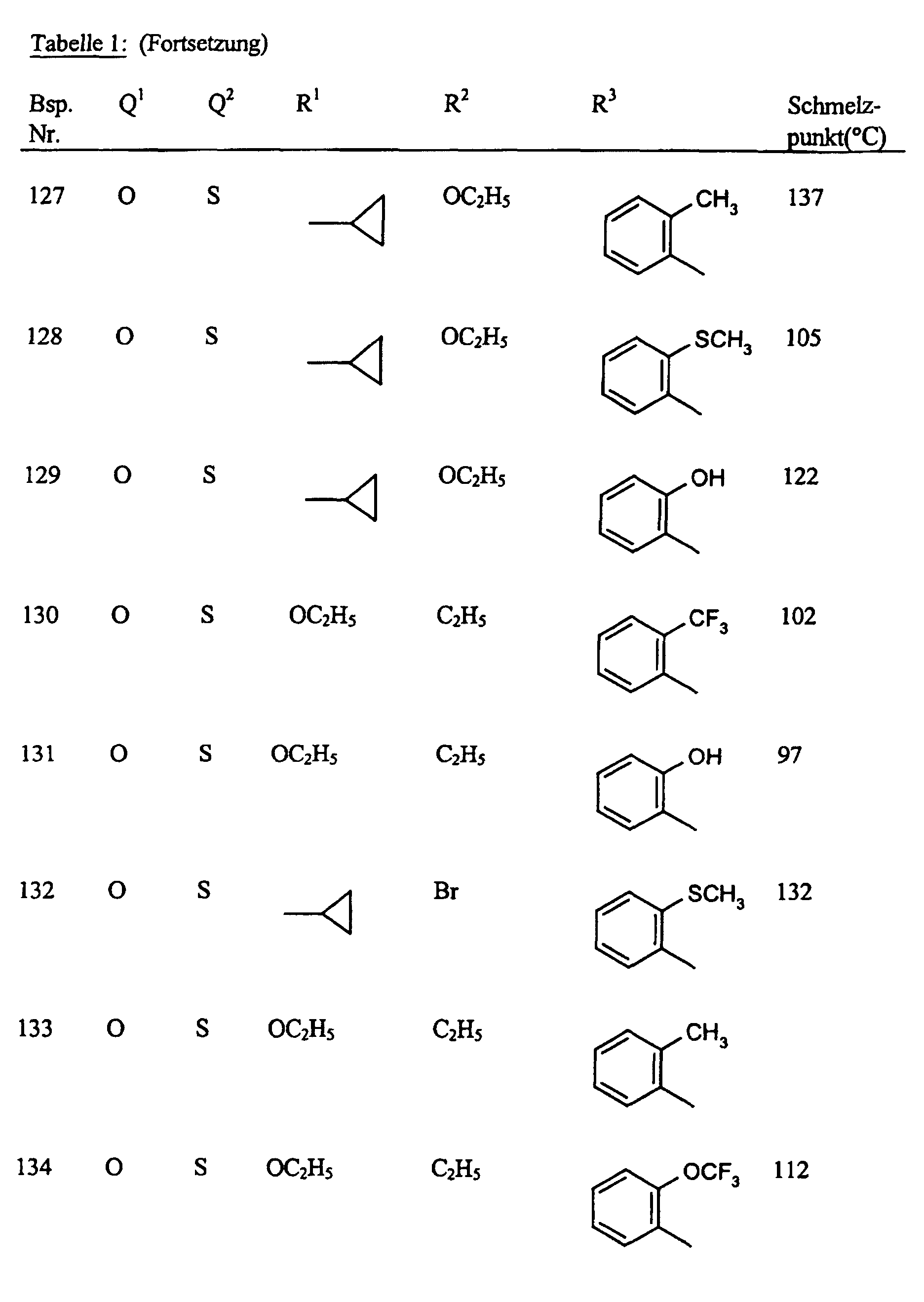

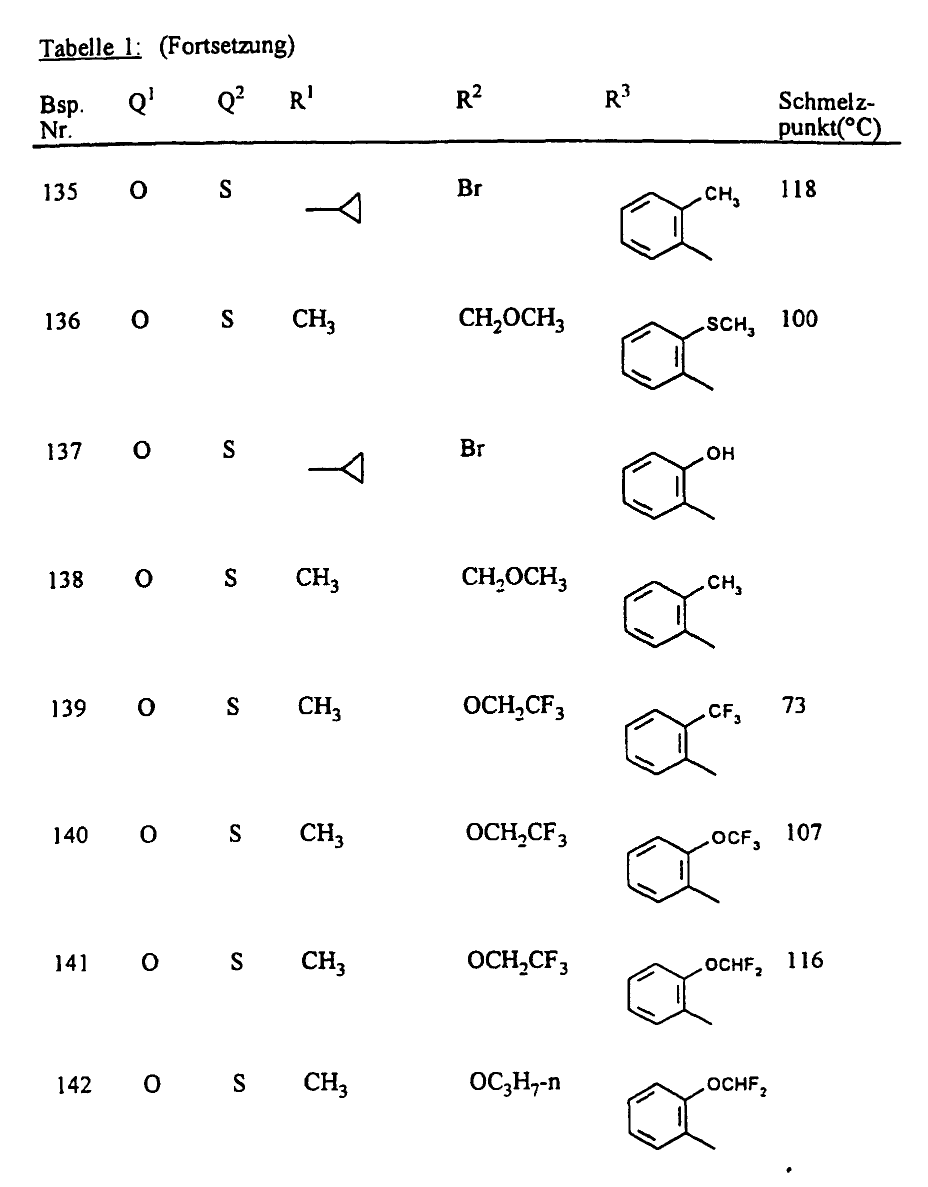

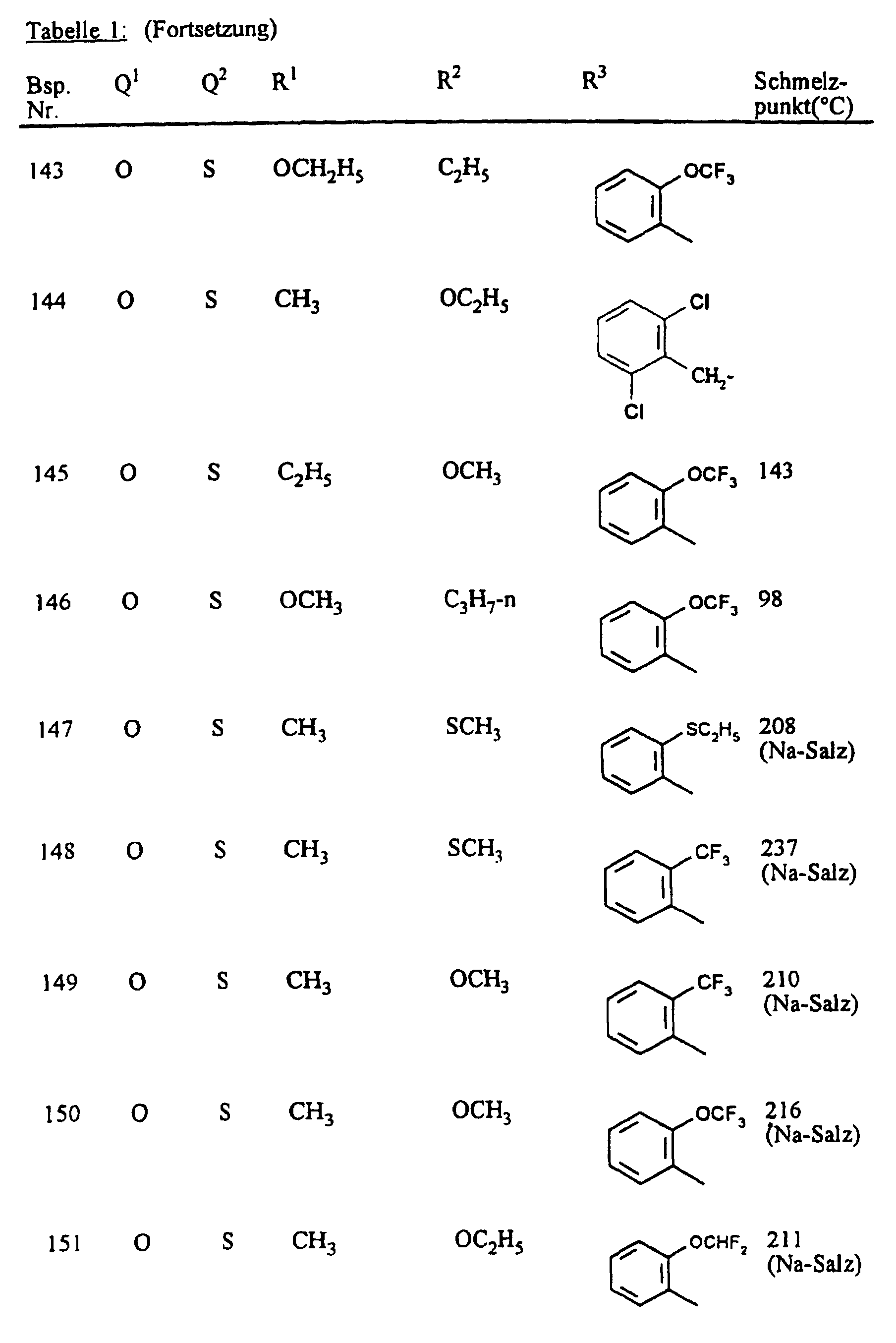

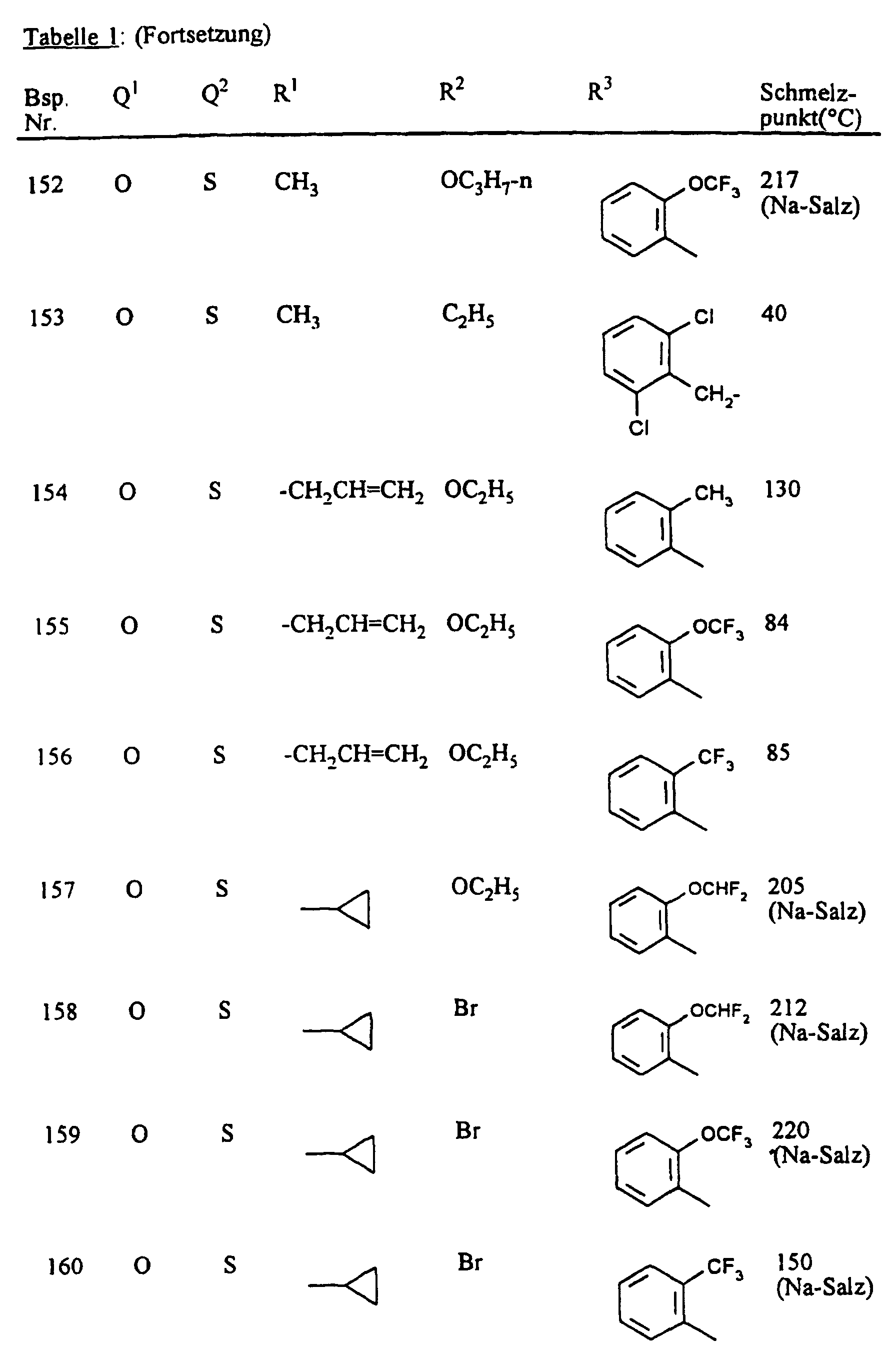

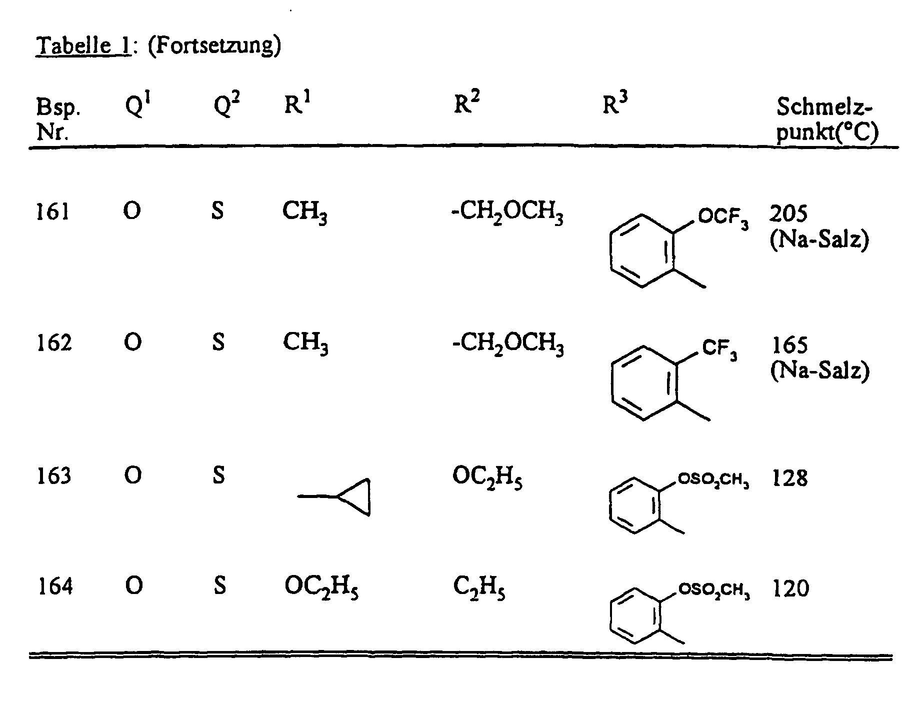

### Ausgangsstoffe der Formel (II):

### Beispiel (II-1)

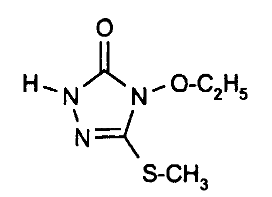

Eine Mischung aus 4,0 g (20 mMol) 4-Ethoxy-5-mercapto-2,4-dihydro-3H-1,2,4-triazol-3-on-Kaliumsalz, 4,3 g (30 mMol) Methyliodid und 50 ml Methanol wird 16 Stunden bei 20°C gerührt und anschließend eingeengt. Der Rückstand wird mit Methylenchlorid/Wasser geschüttelt, die organische Phase abgetrennt, mit Magnesiumsulfat getrocknet und filtriert. Das Filtrat wird eingeengt, der Rückstand mit Diethylether digeriert und das kristallin angefallene Produkt durch Absaugen isoliert.

Man erhält 1,8 g (51% der Theorie) 4-Ethoxy-5-methylthio-2,4-dihydro-3H-1,2,4-triazol-3-on vom Schmelzpunkt 99°C.

Analog Beispiel (II-1) und/oder nach anderen bekannten Verfahren (vgl. die oben angegebenen Publikationen) können beispielsweise auch folgende Verbindungen der Formel (II) hergestellt werden:
4-Methyl-, 4,5-Dimethyl-, 4-Methyl-5-ethyl-, 4-Methyl-5-n-propyl-, 4-Methyl-5-ipropyl-, 4-Methyl-5-n-butyl-, 4-Methyl-5-phenoxy-, 4-Methyl-5-methylthio-, 4-Methyl-5-ethylthio-, 4-Methyl-5-n-propylthio-, 4-Methyl-5-i-propylthio-, 4-Methyl-5-allylthio-, 4-Methyl-5-propargylthio-, 4-Methyl-5-cyclopropyl-, 4-Methyl-5-chlor-, 4-Methyl-5-brom, 4-Methyl-5-methoxy-, 4-Methyl-5-ethoxy-, 4-Methyl-5-npropoxy-, 4-Methyl-5-i-propoxy-, 4-Methyl-5-n-butoxy-, 4-Methyl-5-cyclopropylmethoxy-, 4-Methyl-5-cyclopropylthio-, 4-Methyl-5-trifluorethoxy-, 4-Methyl-5-dimethylamino- und 4-Methyl-5-methylamino- -2,4-dihydro-3H-1,2,4-triazol-3-on;
4-Ethyl-, 4,5-Diethyl-, 4-Ethyl-5-methyl-, 4-Ethyl-5-n-propyl-, 4-Ethyl-5-i-propyl-, 4-Ethyl-5-n-butyl-, 4-Ethyl-5-phenoxy-, 4-Ethyl-5-methylthio-, 4-Ethyl-5-ethylthio-, 4-Ethyl-5-n-propylthio-, 4-Ethyl-5-i-propylthio-, 4-Ethyl-5-allylthio-, 4-Ethyl-5-propargylthio-, 4-Ethyl-5-cyclopropyl-, 4-Ethyl-5-chlor-, 4-Ethyl-5-brom-, 4-Ethyl-5-methoxy-, 4-Ethyl-5-ethoxy-, 4-Ethyl-5-n-propoxy-, 4-Ethyl-5-i-propoxy-, 4-Ethyl-5-n-butoxy-, 4-Ethyl-5-cyclopropylmethoxy-, 4-Ethyl-5-cyclopropylthio-, 4-Ethyl-5-trifluorethoxy-, 4-Ethyl-5-dimethylamino- und 4-Ethyl-5-methylamino- -2,4-dihydro-3H-1,2,4-triazol-3-an;
4-Cyclopropyl-5-methyl-, 4-Cyclopropyl-5-ethyl-, 4-Cyclopropyl-5-n-propyl-, 4-Cyclopropyl-5-i-propyl-, 4-Cyclopropyl-5-n-butyl-, 4-Cyclopropyl-5-phenoxy-, 4-Cyclopropyl-5-methylthio-, 4-Cyclopropyl-5-ethylthio-, 4-Cyclopropyl-5-n-propylthio-, 4-Cyclopropyl-5-i-propylthio-, 4-Cyclopropyl-5-allylthio-, 4-Cyclopropyl-5-propargylthio-, 4-Cyclopropyl-5-cyclopropyl-, 4-Cyclopropyl-5-chlor-, 4-Cyclopropyl-5-brom-, 4-Cyclopropyl-5-methoxy-, 4-Cyclopropyl-5-ethoxy-, 4-Cyclopropyl-5-n-propoxy-, 4-Cyclopropyl-5-i-propoxy-, 4-Cyclopropyl-5-n-butoxy-, 4-Cyclopropyl-5-cyclopropylmethoxy-, 4-Cyclopropyl-5-cyclopropylthio-, 4-Cyclopropyl-5-trifluorethoxy-, 4-Cyclopropyl-5-dimethylamino- und 4-Cyclopropyl-5-methylamino-, -2,4-dihydro-3H-1,2,4-triazol-3-on;
4-Methoxy-, 4,5-Dimethoxy-, 4-Methoxy-5-methyl-, 4-Methoxy-5-ethyl-, 4-Methoxy-5-n-propyl-, 4-Methoxy-5-i-propyl-, 4-Methoxy-5-n-butyl-, 4-Methoxy-5-phenoxy-, 4-Methoxy-5-methylthio-, 4-Methoxy-5-ethylthio-, 4-Methoxy-5-n-propylthio-, 4-Methoxy-5-i-propylthio-, 4-Methoxy-5-allylthio-, 4-Methoxy-5-propargylthio-, 4-Methoxy-5-cyclopropyl-, 4-Methoxy-5-chlor-, 4-Methoxy-5-brom-, 4-Methoxy-5-ethoxy-, 4-Methoxy-5-n-propoxy-, 4-Methoxy-5-i-propoxy-, 4-Methoxy-5-n-butoxy-, 4-Methoxy-5-cyclopropylmethoxy-, 4-Methoxy-5-cyclopropylthio-, 4-Methoxy-5-trifluorethoxy-, 4-Methoxy-5-dimethylamino- und 4-Methoxy-5-methylamino- -2,4-dihydro-3H-1,2,4-triazol-3-on;
4-Ethoxy-, 4,5-Diethoxy-, 4-Ethoxy-5-methyl-, 4-Ethoxy-5-ethyl-, 4-Ethoxy-5-n-propyl-, 4-Ethoxy-5-i-propyl-, 4-Ethoxy-5-n-butyl-, 4-Ethoxy-5-phenoxy-, 4-Ethoxy-5-methylthio-, 4-Ethoxy-5-ethylthio-, 4-Ethoxy-5-n-propylthio-, 4-Ethoxy-5-i-propylthio-, 4-Ethoxy-5-allylthio-, 4-Ethoxy-5-propargylthio-, 4-Ethoxy-5-cyclopropyl-, 4-Ethoxy-5-chlor-, 4-Ethoxy-5-brom-, 4-Ethoxy-5-methoxy-, 4-Ethoxy-5-n-propoxy-, 4-Ethoxy-5-ipropoxy-, 4-Ethoxy-5-n-butoxy-, 4-Ethoxy-5-cyclopropylmethoxy-, 4-Ethoxy-5-cyclopropylthio-, 4-Ethoxy-5-trifluorethoxy-, 4-Ethoxy-5-dimethylamino- und 4-Ethoxy-5-methylamino-, -2,4-dihydro-3H-1,2,4-triazol-3-on;
4-Amino-, 4-Amino-5-methyl-, 4-Amino-5-ethyl-, 4-Amino-5-n-propyl-, 4-Amino-5-ipropyl-, 4-Amino-5-n-butyl-, 4-Amino-5-phenoxy-, 4-Amino-5-methylthio-, 4-Amino-5-ethylthio-, 4-Amino-5-n-propylthio-, 4-Amino-5-i-propylthio-, 4-Amino-5-allylthio-, 4-Amino-5-propargylthio-, 4-Amino-5-cyclopropyl-, 4-Amino-5-chlor-, 4-Amino-5-brom-, 4-Amino-5-methoxy-, 4-Amino-5-ethoxy-, 4-Amino-5-n-propoxy-, 4-Amino-5-i-propoxy-, 4-Amino-5-n-butoxy-, 4-Amino-5-cyclopropylmethoxy-, 4-Amino-5-cyclopropylthio-, 4-Amino-5-trifluorethoxy-, 4-Amino-5-dimethylamino- und 4-Amino-5-methylamino- -2,4-dihydro-3H-1,2,4-triazol-3-on und -2,4-dihydro-3H-1,2,4-triazol-3-thion;
4-Methylamino-, 4-Methylamino-5-methyl-, 4-Methylamino-5-ethyl-, 4-Methylamino-5-n-propyl-, 4-Methylamino-5-i-propyl-, 4-Methylamino-5-n-butyl-, 4-Methylamino-5-phenoxy-, 4-Methylamino-5-methylthio-, 4-Methylamino-5-ethylthio-, 4-Methylamino-5-n-propylthio-, 4-Methylamino-5-i-propylthio-, 4-Methylamino-5-allylthio-, 4-Methylamino-5-propargylthio-, 4-Methylamino-5-cyclopropyl-, 4-Methylamino-5-chlor-, 4-Methylamino-5-brom-, 4-Methylamino-5-methoxy-, 4-Methylamino-5-ethoxy-, 4-Methylamino-5-n-propoxy-, 4-Methylamino-5-i-propoxy-, 4-Methylamino-5-n-butoxy-, 4-Methylamino-5-cyclopropyhnethoxy-, 4-Methylamino-5-cyclopropylthio-, 4-Methylamino-5-trifluorethoxy-, 4-Methylamino-5-dimethylamino- und 4-Methylamino-5-methylamino-, -2,4-dihydro-3H-1,2,4-triazol-3-on;
4-Dimethylamino-, 4-Dimethylamino-5-methyl-, 4-Dimethylamino-5-ethyl-, 4-Dimethylamino-5-n-propyl-, 4-Dimethylamino-5-i-propyl-, 4-Dimethylamino-5-n-butyl-, 4-Dimethylamino-5-phenoxy-, 4-Dimethylamino-5-methylthio-, 4-Dimethylamino-5-ethylthio-, 4-Dimethylamino-5-n-propylthio-, 4-Dimethylamino-5-i-propylthio-, 4-Dimethylamino-5-allylthio-, 4-Dimethylamino-5-propargylthio-, 4-Dimethylamino-5-cyclopropyl, 4-Dimethylamino-5-chlor-, 4-Dimethylamino-5-brom-, 4-Dimethylamino-5-methoxy-, 4-Dimethylamino-5-ethoxy-, 4-Dimethylamino-5-n-propoxy-, 4-Dimethylamino-5-i-propoxy-, 4-Dimethylamino-5-n-butoxy-, 4-Dimethylamino-5-cyclopropylmethoxy-, 4-Dimethylamino-5-cyclopropylthio-, 4-Dimethylamino-5-trifluorethoxy-, 4-Dimethylamino-5-dimethylamino- und 4-Dimethylamino-5-methylamino-, -2,4-dihydro-3H-1,2,4-triazol-3-on.

### Ausgangsstoffe der Formel (IV):

### Beispiel (IV-1)

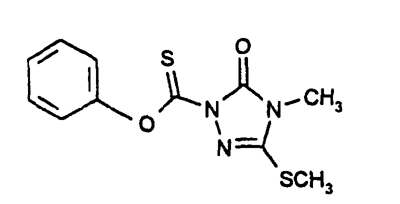

Eine Lösung von 15,0 g (103 mMol) 4-Methyl-5-methylthio-2,4-dihydro-3H-1,2,4-triazol-3-on in 120 ml Methylenchlorid wird bei 20°C mit einer Lösung von 4,6 g Natriumhydroxid und 0,5 g Tetrabutylammoniumbromid in 120 ml Wasser versetzt. Dann wird eine Lösung von 19,6 g (114 mMol) Chlorthioameisensäure-Ophenylester in 100 ml Methylenchlorid bei 20°C tropfenweise dazu gegeben und die Reaktionsmischung wird noch 12 Stunden bei 20°C gerührt. Das kristallin angefallene Produkt wird durch Absaugen isoliert.

Man erhält 25,6 g (88% der Theorie) 4-Methyl-5-methylthio-2-phenoxythiocarbonyl-2,4-dihydro-3H-1,2,4-triazol-3-on vom Schmelzpunkt 242°C.

### Beispiel (IV-2)

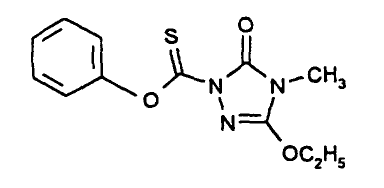

Eine Lösung von 29,6 g (206 mMol) 4-Methyl-5-ethoxy-2,4-dihydro-3H-1,2,4-triazol-3-on in 240 ml Methylenchlorid wird bei 20°C mit einer Lösung von 9,2 g Natriumhydroxid und 1 g Tetrabutylammoniumbromid in 240 ml Wasser versetzt. Dann wird eine Lösung von 39,2 g (228 mMol) Chlorthioameisensäure-O-phenylester in 200 ml Methylenchlorid bei 20°C tropfenweise dazu gegeben und die Reaktionsmischung wird noch 12 Stunden bei 20°C gerührt. Die organische Phase wird dann abgetrennt und die wässrige Phase mit 100 ml Methylenchlorid nachextrahiert. Die vereinigten organischen Phasen werden mit 200 ml Wasser gewaschen, mit Magnesiumsulfat getrocknet und filtriert. Das Filtrat wird im Wasserstrahlvakuum eingeengt, der Rückstand mit Diethylether verrührt und das kristallin angefallene Produkt durch Absaugen isoliert.

Man erhält 44,3 g (77% der Theorie) 4-Methyl-5-ethoxy-2-phenoxythiocarbonyl-2,4-dihydro-3H-1,2,4-triazol-3-on vom Schmelzpunkt 123°C.

Analog zu den Beispielen (IV-1) und (IV-2) können beispielsweise auch die in der nachstehenden Tabelle 2 aufgeführten Verbindungen der Formel (IV) hergestellt werden.

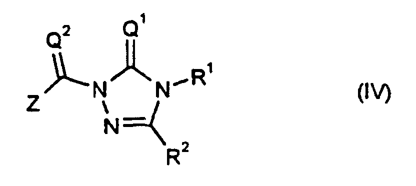

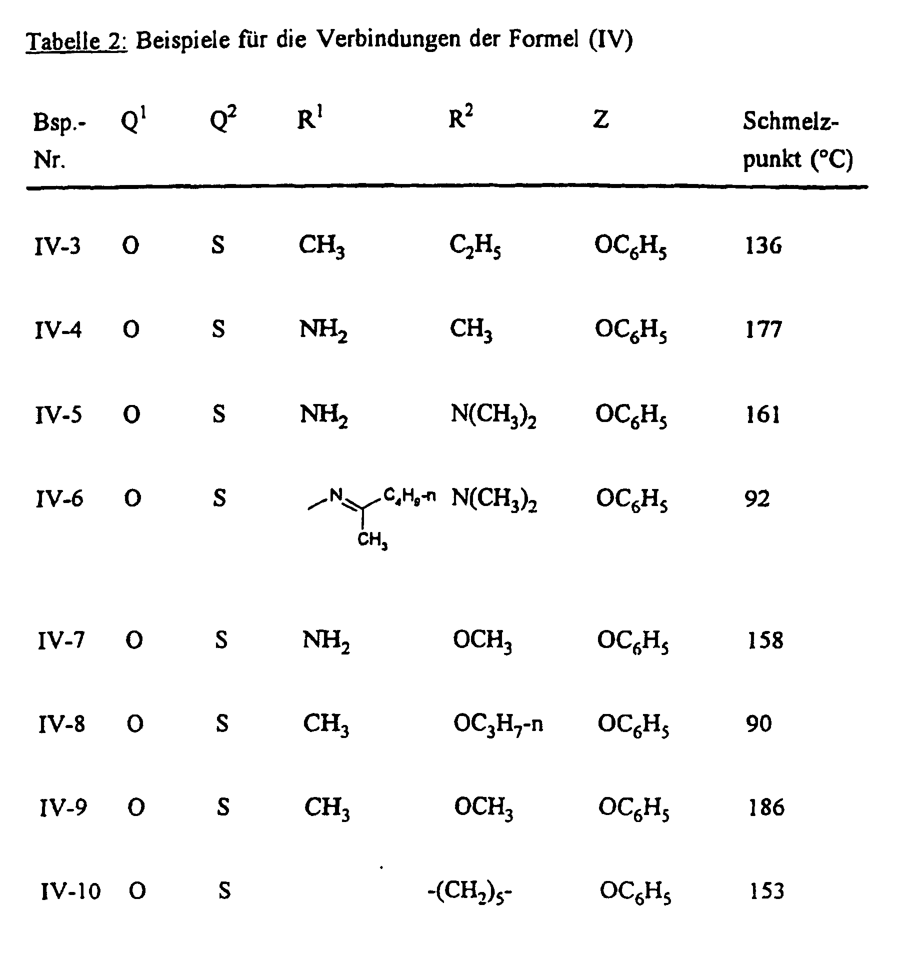

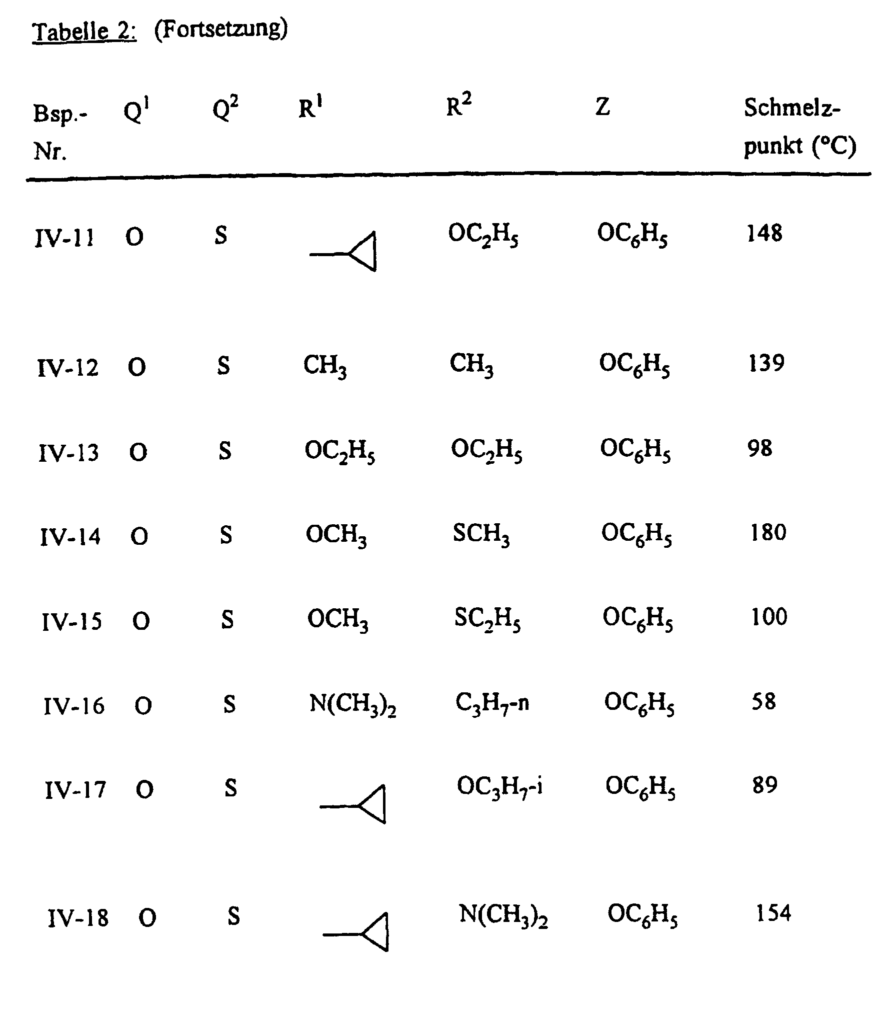

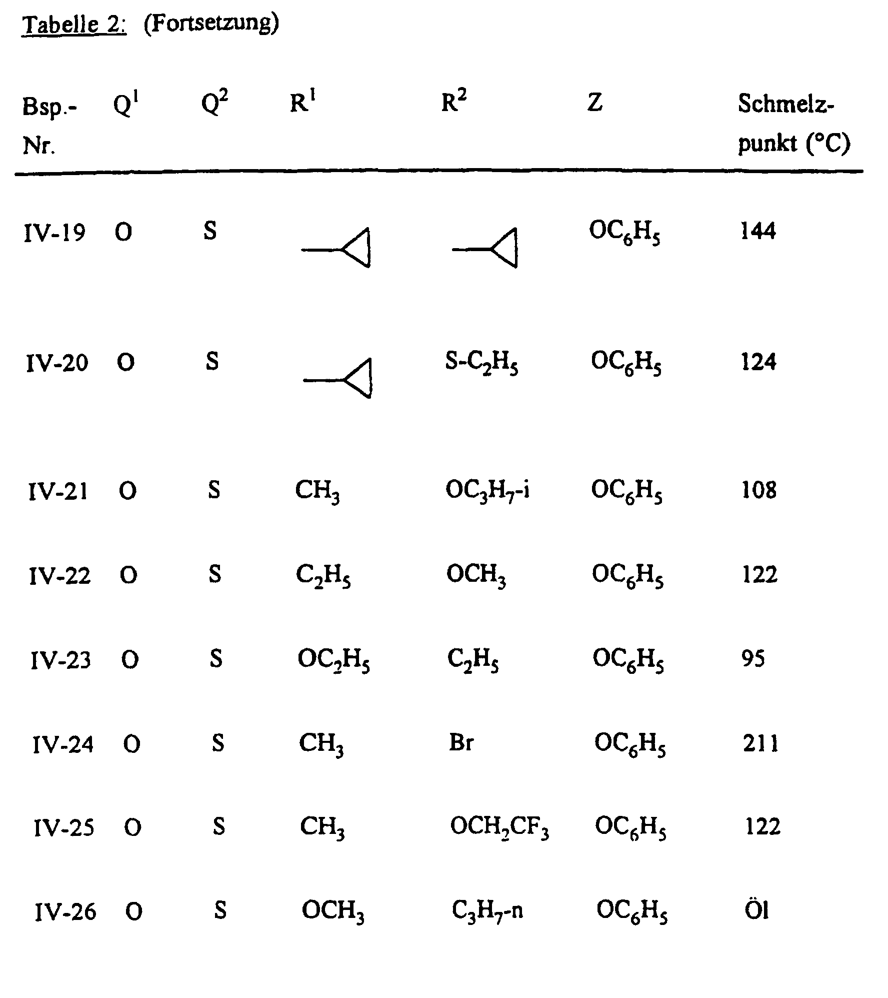

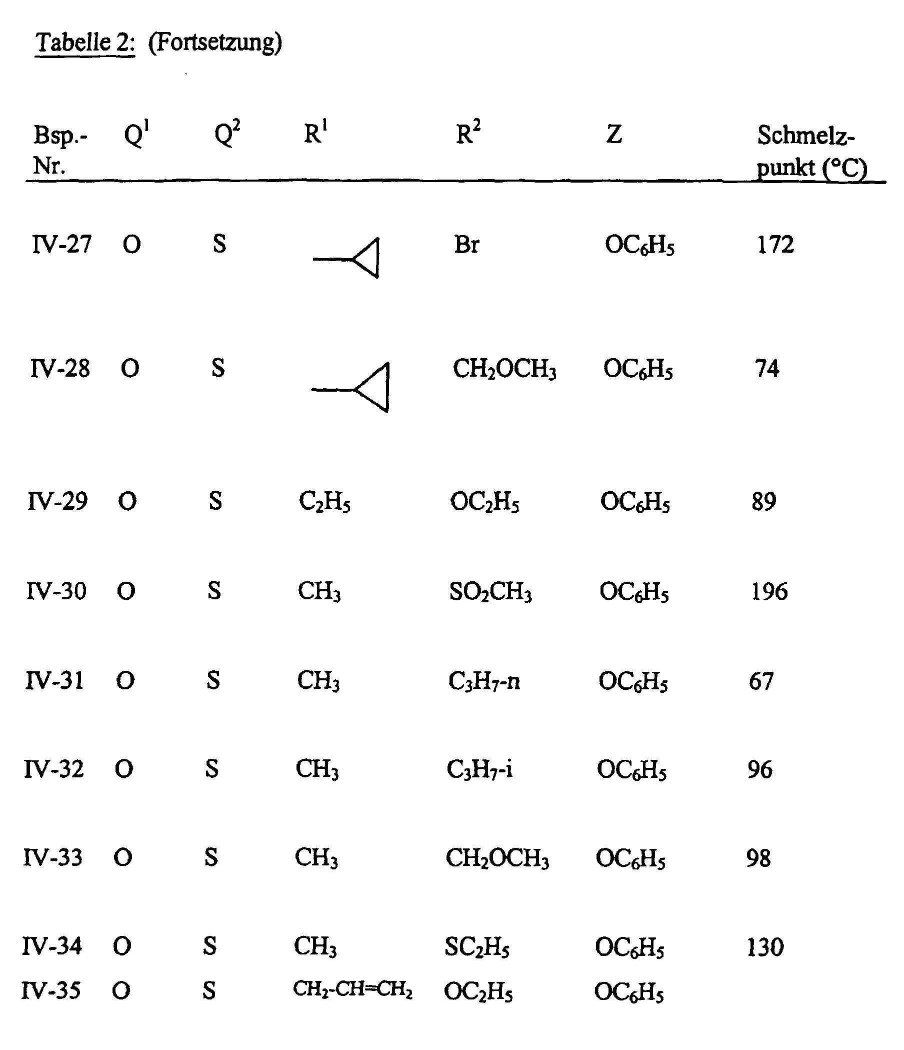

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % | = keine Wirkung (wie unbehandelte Kontrolle) |
| 100 % | = totale Vernichtung |

In diesem Test zeigen beispielsweise die Verbindungen gemäß den Herstellungsbeispielen 3, 23, 27 und 28 bei teilweise sehr guter Verträglichkeit gegenüber Kulturpflanzen, wie z.B. Baumwolle, sehr starke Wirkung gegen Unkräuter.

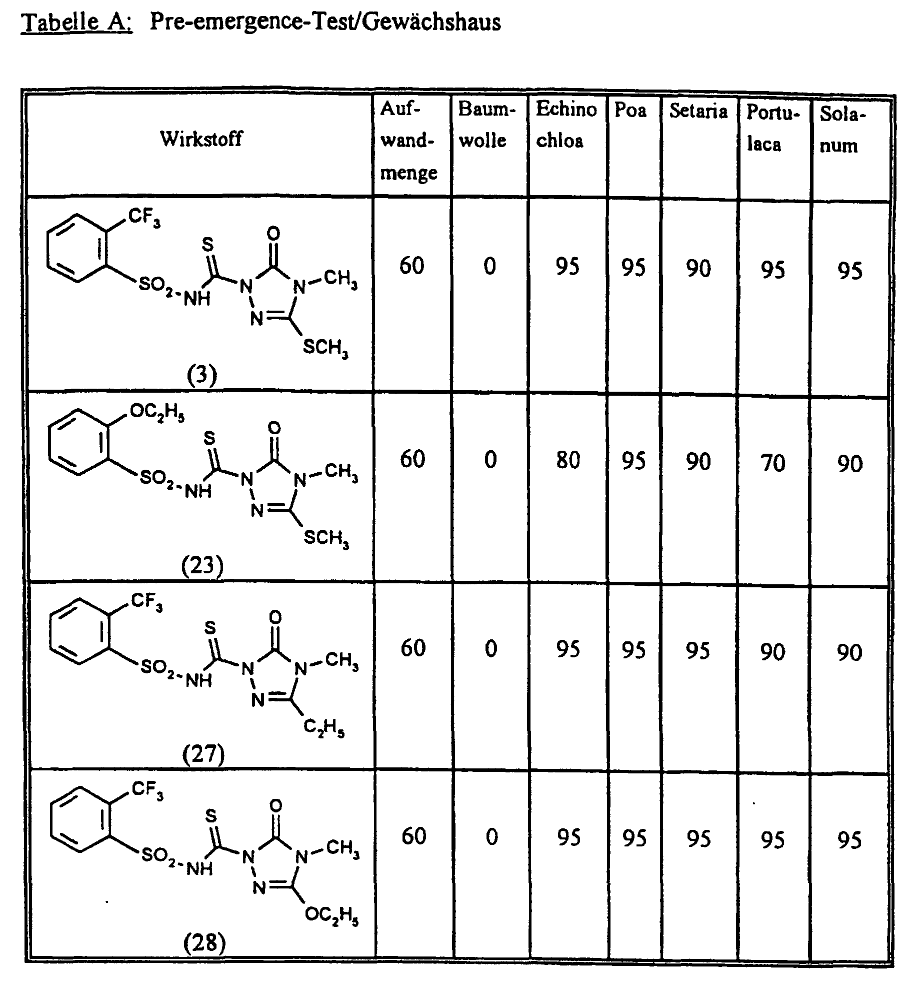

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % | = keine Wirkung (wie unbehandelte Kontrolle) |
| 100 % | = totale Vernichtung |

In diesem Test zeigen beispielsweise die Verbindungen gemäß den Herstellungsbeispielen 3, 15 und 28 bei guter Verträglichkeit gegenüber Kulturpflanzen, wie z.B. Weizen, sehr starke Wirkung gegen Unkräuter.

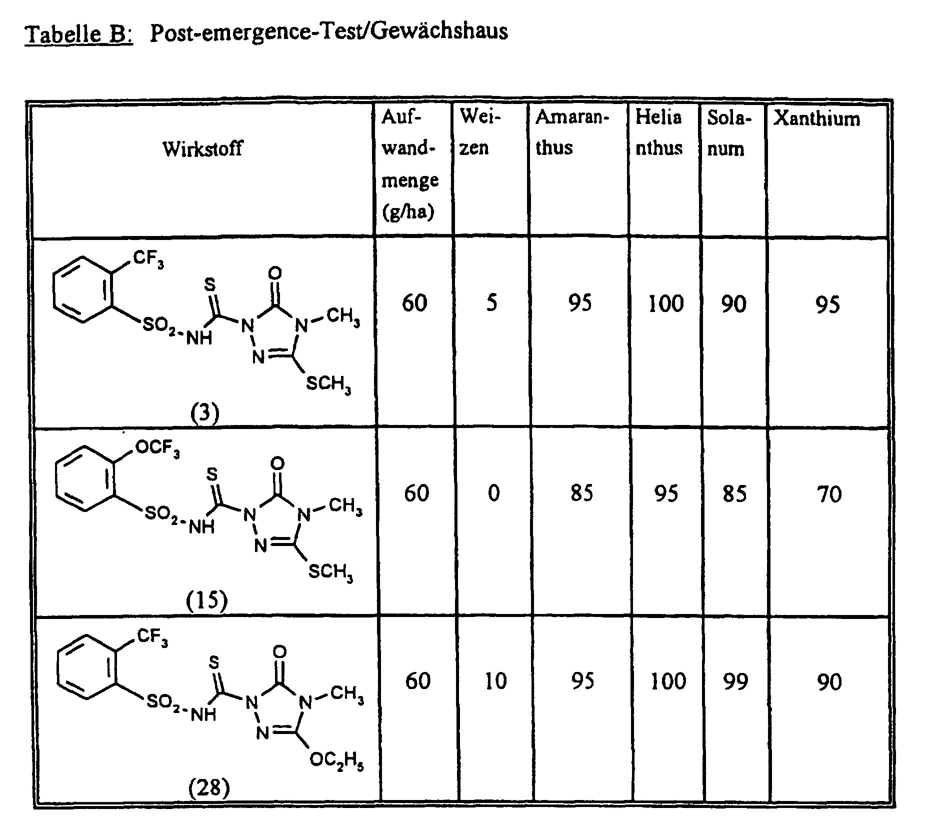

## Patentansprüche

1. Sulfonylaminothiocarbonyl-triazolinone der allgemeinen Formel (I) 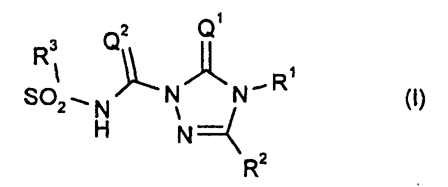 in welcher
Q¹ für Sauerstoff steht,
Q² für Schwefel steht,
R¹ für Wasserstoff, Hydroxy, Amino, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxy-carbonyl substituiertes C₁-C₆-Alkoxy, C₁-C₆-Alkylamino oder C₁-C₆-Alkanoylamino, für C₃-C₆-Alkenyloxy, für Di-(C₁-C₄-alkyl)-amino, für jeweils gegebenenfalls durch Fluor, Chlor, Brom, Cyano und/oder C₁-C₄-Alkyl substituiertes C₃-C₆-Cycloalkyl, C₃-C₆-Cycloalkylamino oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl und/oder C₁-C₄-Alkoxy substituiertes Phenyl oder Phenyl-C₁-C₄-alkyl steht,
R² für Wasserstoff, Hydroxy, Mercapto, Amino, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxycarbonyl substituiertes C₁-C₆-Alkoxy, C₁-C₆-Alkylthio, C₁-C₆-Alkylamino oder C₁-C₆-Alkanoylamino, für C₃-C₆-Alkenyloxy, C₃-C₆-Alkinyloxy, C₃-C₆-Alkenylthio, C₃-C₆-Alkinylthio, C₃-C₆-Alkenylamino oder C₃-C₆-Alkinylamino, für Di-(C₁-C₄-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes C₃-C₆-Cycloalkyl, C₅-C₆-Cycloalkenyl, C₃-C₆-Cycloalkyloxy, C₃-C₆-Cycloalkylthio, C₃-C₆-Cycloalkylamino, C₃-C₆-Cycloalkyl-C₁-C₄-alkyl, C₃-C₆-Cycloalkyl-C₁-C₄-alkoxy, C₃-C₆-Cycloalkyl-C₁-C₄alkylthio oder C₃-C₆-Cycloalkyl-C₁-C₄-alkylamino, oder für jeweils gegebenenfalls durch Fluor, Chlor, Brom, Cyano, Nitro, C₁-C₄-Alkyl, Trifluormethyl und/oder C₁-C₄-Alkoxy substituierte substituiertes Phenyl, Phenyl-C₁-C₄-alkyl, Phenoxy, Phenyl-C₁-C₄-alkoxy, Phenylthio, Phenyl-C₁-C₄-alkylthio, Phenylamino oder Phenyl-C₁-C₄-alkylamino steht, oder
R¹ und R² zusammen für gegebenenfalls verzweigtes Alkandiyl mit 3 bis 11 Kohlenstoffatomen stehen, und
R³ für die Gruppierung 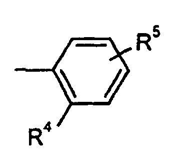 steht,
worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)-amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆--Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxycarbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxycarbonyl, C₁-C₄-Alkylthio, C₁-C₄-Alkylsulfinyl oder C₁-C₄-Alkylsulfonyl substituiert ist), für C₂-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkylcarbonyl, C₁-C₄-Alkoxy-carbonyl, C₁-C₄-Alkylaminocarbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxy- carbonyl substituiert ist) steht,
R⁴ und/oder R⁵ weiterhin für Phenyl oder Phenoxy, für C₁-C₄-Alkylcarbonylamino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁸ stehen, wobei
R⁸ für Wasserstoff, C₁-C₆-Alkyl, C₃-C₆-Cycloalkyl, C₁-C₆-Alkoxy, C₃-C₆-Cycloalkoxy, C₃-C₆-Alkenyloxy, C₁-C₄-Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkylsulfonyloxy, Di-(C₁-C₄-alkyl)-aminosulfonylamino oder für den Rest-CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenyloxy, C₃-C₆-Alkinyloxy oder Benzyloxy, für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht, oder
weiterhin
R³ für den Rest 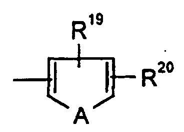 steht,
worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), C₁-C₄-Alkylthio, C₁-C₄-Alkylsulfinyl oder C₁-C₄-Alkylsulfonyl (welche jeweils gegebenenfalls durch Fluor und/oder Chlor substituiert sind), Di-(C₁-C₄-alkyl)-amino-sulfonyl, C₁-C₄-Alkoxy-carbonyl oder Dimethylaminocarbonyl stehen, und
A für Schwefel steht,
sowie deren Natrium-, Kalium-, Magnesium-, Calcium-, Ammonium-, C₁-C₄-Alkyl-ammonium-, Di-(C₁-C₄-alkyl)-ammonium-, Tri-(C₁-C₄alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze.

2. Verbindungen der Formel (I) gemäß Anspruch 1, **dadurch gekennzeichnet, dass** darin
Q¹ für Sauerstoff steht,
Q² für Schwefel steht,
R¹ für Wasserstoff, Hydroxy, Amino, für C₃-C₈-Alkylidenamino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht,
R² für Wasserstoff, Hydroxy, Mercapto, Amino, Fluor, Chlor, Brom, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Propenyloxy, Butenyloxy, Propinyloxy, Butinyloxy, Propenylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Trifluormethyl und/oder Methoxy substituiertes Phenyl, Benzyl, Phenoxy, Benzyloxy, Phenylthio, Benzylthio, Phenylamino oder Benzylamino steht, oder
R¹ und R² zusammen für gegebenenfalls verzweigtes Alkandiyl mit 3 bis 11 Kohlenstoffatomen stehen, und
R³ für den Rest 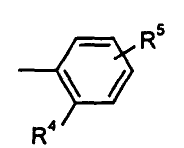 steht,
worin
R⁴ für Fluor, Chlor, Brom, für jeweils gegebenenfalls durch Fluor und/oder Chlor substituiertes Methyl, Ethyl, n- oder i-Propyl, für jeweils gegebenenfalls durch Fluor, Chlor, Methoxy oder Ethoxy substituiertes Methoxy, Ethoxy, n- oder i-Propoxy, Methylthio, Ethylthio, n- oder i-Propylthio, Methylsulfinyl, Ethylsulfinyl, n-oder i-Propylsulfinyl, Methylsulfonyl, Ethylsulfonyl, n- oder i-Propylsulfonyl, Dimethylaminosulfonyl, Diethylaminosulfonyl, N-Methoxy-N-methylaminosulfonyl, Methoxyaminosulfonyl, Phenyl, Phenoxy, oder für jeweils gegebenenfalls durch Fluor, Chlor, Methoxy oder Ethoxy substituiertes Methoxycarbonyl, Ethoxycarbonyl, n- oder i-Propoxycarboriyl steht und
R⁵ für Wasserstoff, Methyl, Ethyl, Fluor, Chlor oder Brom steht;
weiterhin
R³ für den Rest 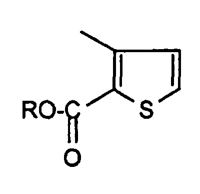 steht,
worin
R für Methyl, Ethyl, n- oder i-Propyl steht.

3. Verfahren zur Herstellung von Sulfonylaminothiocarbonyltriazolinonen der Formel (I) und deren Salzen gemäß Anspruch 1, **dadurch gekennzeichnet, dass** man
(a) Triazolinone der allgemeinen Formel (II) 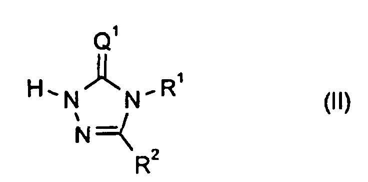 in welcher
Q¹, R¹ und R² die in Anspruch 1 angegebene Bedeutung haben,
mit Sulfonylisothiocyanaten der allgemeinen Formel (III)
R³-SO₂-N=C=Q² (III)
in welcher
Q² und R³ die in Anspruch 1 angegeben Bedeutung haben,
gegebenenfalls in Gegenwart eines Reaktionshilfsmittels und gegebenenfalls in Gegenwart eines Verdünnungsmittels umsetzt,
oder dass man
(b) Triazolinon-Derivate der allgemeinen Formel (IV) 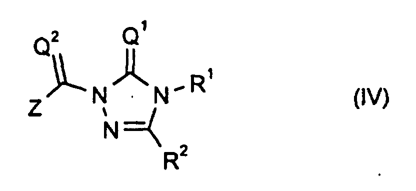 in welcher
Q¹, Q², R¹ und R² die in Anspruch 1 angegebene Bedeutung haben und
Z für Halogen, Alkoxy, Aryloxy oder Arylalkoxy steht,
mit Sulfonsäureamiden der allgemeinen Formel (V)
R³-SO₂-NH₂ (V)
in welcher
R³ die in Anspruch 1 angegebene Bedeutung hat,
gegebenenfalls in Gegenwart eines Säureakzeptors und gegebenenfalls in Gegenwart eines Verdünnungsmittels umsetzt,
oder dass man
(c) Triazolinone der allgemeinen Formel (II) 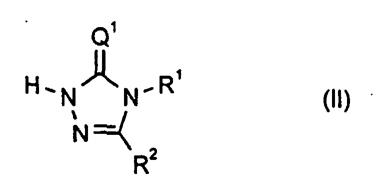 in welcher
Q¹, R¹ und R² die in Anspruch 1 angegebene Bedeutung haben,
mit Sulfonsäureamid-Derivaten der allgemeinen Formel (VI)
R³-SO₂-NH-CQ²-Z (VI)
in welcher
Q² und R³ die in Anspruch 1 angegebene Bedeutung haben und
Z für für Halogen, Alkoxy, Aryloxy oder Arylalkoxy steht,
gegebenenfalls in Gegenwart eines Säureakzeptors und gegebenenfalls in Gegenwart eines Verdünnungsmittels umsetzt,
oder dass man
(d) Triazolinone der allgemeinen Formel (II) 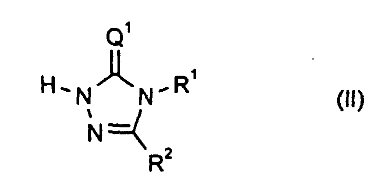 in welcher
Q¹, R¹ und R² die in Anspruch 1 angegebene Bedeutung haben,
mit Sulfonsäurehalogeniden der allgemeinen Formel (VII)
R³-SO₂-X (VII)
in welcher
R³ die in Anspruch 1 angegebene Bedeutung hat und
X für Halogen steht,
und Metallthiocyanaten der allgemeinen Formel (VIII)
MQ²CN (VIII)
in welcher
Q² die in Anspruch 1 angegebene Bedeutung hat und
M für ein Alkalimetall oder ein Erdalkalimetalläqivalent steht,
gegebenenfalls in Gegenwart eines Reaktionshilfsmittels und gegebenenfalls in Gegenwart eines Verdünnungsmittels umsetzt,
und gegebenenfalls die nach Verfahren (a), (b), (c) oder (d) erhaltenen Verbindungen der Formel (I) nach üblichen Methoden in Salze überführt.

4. Herbizide Mittel, **gekennzeichnet durch** einen Gehalt an mindestens einer Verbindung der Formel (I) oder einem ihrer Salze gemäß Anspruch 1.

5. Verwendung von Verbindungen der allgemeinen Formel (I) oder deren Salzen gemäß Anspruch 1 zur Bekämpfung von unerwünschtem Pflanzenwachstum.

6. Verfahren zur Bekämpfung von Unkräutern, **dadurch gekennzeichnet, dass** man Verbindungen der allgemeinen Formel (I) oder deren Salze gemäß Anspruch 1 auf die Unkräuter oder ihren Lebensraum einwirken läßt.

7. Verfahren zur Herstellung von herbiziden Mitteln, **dadurch gekennzeichnet, dass** man Verbindungen der allgemeinen Formel (I) oder deren Salze gemäß Anspruch 1 mit Streckmitteln und/oder oberflächenaktiven Mitteln vermischt.

8. Triazolinon-Derivate der allgemeinen Formel (IV) 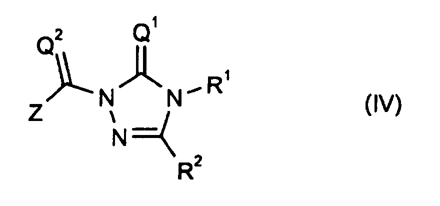 in welcher
Q¹ für Sauerstoff steht,
Q² für Schwefel steht,
R¹ für Wasserstoff, Hydroxy, für gegebenenfalls durch Fluor, Chlor, Brom, Cyano, C₁-C₄-Alkoxy, C₁-C₄-Alkyl-carbonyl oder C₁-C₄-Alkoxycarbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für gegebenenfalls durch Fluor, Chlor, Cyano, C₁-C₄-Alkoxy oder C₁-C₄-Alkoxy-carbonyl substituiertes C₁-C₆-Alkoxy, für C₃-C₆-Alkenyloxy, für jeweils gegebenenfalls durch Fluor, Chlor, Brom, Cyano und/oder C₁-C₄-Alkyl substituiertes C₃-C₆-Cycloalkyl oder C₃-C₆-Cycloalkyl-C₁-C₄-alkyl, steht,
R² für Wasserstoff, Hydroxy, Mercapto, Amino, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxycarbonyl substituiertes C₁-C₆-Alkoxy, C₁-C₆-Alkylthio, C₁-C₆-Alkylamino oder C₁-C₆-Alkanoylamino, für C₃-C₆-Alkenyloxy, C₃-C₆-Alkinyloxy, C₃-C₆-Alkenylthio, C₃-C₆-Alkinylthio, C₃-C₆-Alkenylamino oder C₃-C₆-Alkinylamino, für Di-(C₁-C₄-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes C₃-C₆-Cycloalkyl, C₅-C₆-Cycloalkenyl, C₃-C₆-Cycloalkyloxy, C₃-C₆-Cycloalkylthio, C₃-C₆-Cycloalkylamino, C₃-C₆-Cycloalkyl-C₁-C₄-alkyl, C₃-C₆-Cycloalkyl-C₁-C₄-alkoxy, C₃-C₆-Cycloalkyl-C₁-C₄alkylthio oder C₃-C₆-Cycloalkyl-C₁-C₄-alkylamino, oder für jeweils gegebenenfalls durch Fluor, Chlor, Brom, Cyano, Nitro, C₁-C₄-Alkyl, Trifluormethyl und/oder C₁-C₄-Alkoxy substituierte substituiertes Phenyl, Phenyl-C₁-C₄-alkyl, Phenoxy, Phenyl-C₁-C₄-alkoxy, Phenylthio, Phenyl-C₁-C₄-alkylthio, Phenylamino oder Phenyl-C₁-C₄-alkylamino steht, oder
R¹ und R² zusammen für gegebenenfalls verzweigtes Alkandiyl mit 3 bis 11 Kohlenstoffatomen stehen, und
Z für Fluor, Chlor, Brom, Methoxy, Ethoxy, Benzyloxy oder Phenoxy steht.

## Claims

1. Sulphonylaminothiocarbonyl-triazolinethiones of the general formula (I) 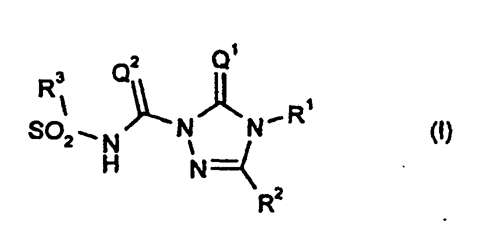 in which
Q¹ represents oxygen,
Q² represents sulphur,
R¹ represents hydrogen, hydroxyl or amino, or represents C₂-C₁₀-alkylideneamino or represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, or represents respectively optionally fluorine-, chlorine- and/or bromine-substituted C₂-C₆-alkenyl or C₂-C₆-alkinyl, or represents respectively optionally fluorine-, chlorine-, cyano-, C₁-C₄-alkoxy- or C₁-C₄-alkoxy-carbonyl-substituted C₁-C₆-alkoxy, C₁-C₆-alkylamino or C₁-C₆-alkarioylamino, or represents C₃-C₆-alkenyloxy, or represents di-(C₁-C₄-alkyl)-amino, or represents respectively optionally fluorine-, chlorine-, bromine-, cyano- and/or C₁-C₄-alkyl-substituted C₃-C₆-cycloalkyl, C₃-C₆-cycloalkylamino or C₃-C₆-cycloalkyl-C₁-C₄-alkyl, or represents respectively optionally fluorine-, chlorine-, bromine-, cyano-, nitro-, C₁-C₄-alkyl-, trifluoromethyl- and/or C₁-C₄-alkoxy-substituted phenyl or phenyl-C₁-C₄-alkyl,
R² represents hydrogen, hydroxyl, mercapto, amino, fluorine, chlorine, bromine or iodine, or represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, or represents respectively optionally fluorine-, chlorine- and/or bromine-substituted C₂-C₆-alkenyl or C₂-C₆-alkinyl, or represents respectively optionally fluorine-, chlorine-, cyano-, C₁-C₄-alkoxy- or C₁-C₄-alkoxy-carbonyl-substituted C₁-C₆-alkoxy, C₁-C₆-alkylthio, C₁-C₆-alkylamino or C₁-C₆-alkanoylamino, or represents C₃-C₆-alkenyloxy, C₃-C₆-alkinyloxy, C₃-C₆-alkenylthio, C₃-C₆-alkinylthio, C₃-C₆-alkenylamino or C₃-C₆-alkinylamino, or represents di-(C₁-C₄-alkyl)-amino, or represents respectively optionally methyl- and/or ethyl-substituted aziridino, pyrrolidino, piperidino or morpholino, or represents respectively optionally fluorine-, chlorine-, bromine-, cyano- and/or C₁-C₄-alkyl-substituted C₃-C₆-cycloalkyl, C₅-C₆-cycloalkenyl, C₃-C₆-cycloalkyloxy, C₃-C₆-cycloalkylthio, C₃-C₆-cycloalkylamino, C₃-C₆-cycloalkyl-C₁-C₄-alkyl, C₃-C₆-cycloalkyl-C₁-C₄-alkoxy, C₃-C₆-cycloalkyl-C₁-C₄-alkylthio or C₃-C₆-cycloalkyl-C₁-C₄-alkylamino, or represents respectively optionally fluorine-, chlorine-, bromine-, cyano-, nitro-, C₁-C₄-alkyl-, trifluoromethyl- and/or C₁-C₄-alkoxy-substituted phenyl, phenyl-C₁-C₄-alkyl, phenoxy, phenyl-C₁-C₄-alkoxy, phenylthio, phenyl-C₁-C₄-alkylthio, phenylamino or phenyl-C₁-C₄-alkylamino, or
R¹ and R² together represent optionally branched alkanediyl having 3 to 11 carbon atoms, and
R³ represents the grouping 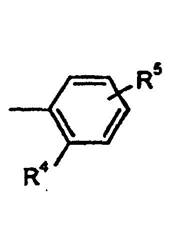 in which
R⁴ and R⁵ are identical or different and each represent hydrogen, fluorine, chlorine, bromine, iodine, nitro, C₁-C₆-alkyl (which is optionally substituted by fluorine, chlorine, bromine, cyano, carboxyl, C₁-C₄-alkoxycarbonyl, C₁-C₄-alkylamino-carbonyl, di-(C₁-C₄-alkyl)-amino-carbonyl, hydroxyl, C₁-C₄-alkoxy, formyloxy, C₁-C₄-alkyl-carbonyloxy, C₁-C₄-allcoxy-carbonyloxy, C₁-C₄-alkylamino-carbonyloxy, C₁-C₄-alkylthio, C₁-C₄-alkylsulphinyl, C₁-C₄-alkylsulphonyl, di-(C₁-C₄-alkyl)-aminosulphonyl, C₃-C₆-cycloalkyl or phenyl), or represent C₂-C₆-alkenyl (which is optionally substituted by fluorine, chlorine, bromine, cyano, C₁-C₄-alkoxy-carbonyl, carboxyl or phenyl), or represent C₂-C₆-alkinyl (which is optionally substituted by fluorine, chlorine, bromine, cyano, C₁-C₄-alkoxy-carbonyl, carboxyl or phenyl), or represent C₁-C₄-alkoxy (which is optionally substituted by fluorine, chlorine, bromine, cyano, carboxyl, C₁-C₄-alkoxy-carbonyl, C₁-C₄-alkoxy, C₁-C₄-alkylthio, C₁-C₄-alkylsulphinyl or C₁-C₄-alkylsulphonyl), or represent C₁-C₄-alkylthio (which is optionally substituted by fluorine, chlorine, bromine, cyano, carboxyl, C₁-C₄-alkoxy-carbonyl, C₁-C₄-alkylthio, C₁-C₄-alkylsulphinyl or C₁-C₄-alkylsulphonyl), or represent C₂-C₆-alkenyloxy (which is optionally substituted by fluorine, chlorine, bromine, cyano or C₁-C₄-allcoxy-carbonyl), or represent C₂-C₆-alkenylthio (which is optionally substituted by fluorine, chlorine, bromine, cyano, nitro, C₁-C₃-alkylthio or C₁-C₄-alkoxy-carbonyl), C₃-C₆-alkinyloxy, C₃-C₆-alkinylthio, or represent the radical -S(O)ₚ-R⁶ in which
p represents the numbers 1 or 2 and
R⁶ represents C₁-C₄-alkyl (which is optionally substituted by fluorine, chlorine, bromine, cyano or C₁-C₄-alkoxy-carbonyl), C₃-C₆-alkenyl, C₃-C₆-alkinyl, C₁-C₄-alkoxy, C₁-C₄-alkoxy-C₁-C₄-alkylamino, C₁-C₄-alkylamino, di-(C₁-C₄-alkyl)-amino or phenyl, or represent the radical - NHOR⁷ in which
R⁷ represents C₁-C₁₂-alkyl (which is optionally substituted by fluorine, chlorine, cyano, C₁-C₄-alkoxy, C₁-C₄-alkylthio, C₁-C₄-alkylsulphinyl, C₁-C₄-allcylsulphonyl, C₁-C₄-alkyl-carbonyl, C₁-C₄-alkoxy-carbonyl, C₁-C₄-alkylamino-carbonyl or di-(C₁-C₄-alkyl)-amino-carbonyl), or represents C₃-C₆-alkenyl (which is optionally substituted by fluorine, chlorine or bromine), C₃-C₆-alkinyl, C₃-C₆-cycloalkyl, C₃-C₆-cycloalkyl-C₁-C₂-alkyl or phenyl-C₁-C₂-alkyl (which is optionally substituted by fluorine, chlorine, nitro, cyano, C₁-C₄-alkyl, C₁-C₄-alkoxy or C₁-C₄-alkoxy-carbonyl), or represents benzhydryl or represents phenyl (which is optionally substituted by fluorine, chlorine, nitro, cyano, C₁-C₄-alkyl, trifluoromethyl, C₁-C₄-alkoxy, C₁-C₂-fluoroalkoxy, C₁-C₄-alkylthio, trifluoromethylthio or C₁-C₄-alkoxy-carbonyl),
R⁴ and R⁵ further represent phenyl or phenoxy, or represent C₁-C₄-alkyl-carbonylamino, C₁-C₄-alkoxy-carbonylamino, C₁-C₄-alkylamino-carbonyl-amino or di-(C₁-C₄-alkyl)-amino-carbonylamino, or represent the radical -CO-R⁸ in which
R⁸ represents hydrogen, C₁-C₆-alkyl, C₃-C₆-cycloalkyl, C₁-C₆-alkoxy, C₃-C₆-cycloalkoxy, C₃-C₆-alkenyloxy, C₁-C₄-alkylthio, C₁-C₄-alkylamino, C₁-C₄-alkoxyamino, C₁-C₄-alkoxy-C₁-C₄-alkyl-amino or di-(C₁-C₄-alkyl)-amino (each of which is optionally - if appropriate - substituted by fluorine and/or chlorine),
R⁴ and/or R⁵ further represent trimethylsilyl, thiazolinyl, C₁-C₄-alkyl-sulphonyloxy or di-(C₁-C₄-alkyl)-aminosulphonylamino, or represent the radical -CH=N-R⁹ in which
R⁹ represents optionally fluorine-, chlorine-, cyano-, carboxyl-, C₁-C₄-alkoxy-, C₁-C₄-alkylthio-, C₁-C₄-alkylsulphinyl- or C₁-C₄-alkylsulphonyl-substituted C₁-C₆-alkyl, or represents optionally fluorine- or chlorine-substituted benzyl, or represents optionally fluorine- or chlorine-substituted C₃-C₆-alkenyl or C₃-C₆-alkinyl, or represents optionally fluorine-, chlorine-, bromine-, C₁-C₄-alkyl-, C₁-C₄-alkoxy-, trifluoromethyl-, trifluoromethoxy- or trifluoromethylthio-substituted phenyl, or represents optionally fluorine- and/or chlorine-substituted C₁-C₆-alkoxy, C₃-C₆-alkenyloxy, C₃-C₆-alldnyloxy or benzyloxy, or represents amino, C₁-C₄-alkylamino, di-(C₁-C₄-alkyl)-amino, phenylamino, C₁-C₄-alkyl-carbonyl-amino, C₁-C₄-alkoxy-carbonylamino or C₁-C₄-alkyl-sulphonylamino, or represents optionally fluorine-, chlorine-, bromine- or methyl-substituted phenylsulphonylamino , or
furthermore,
R³ represents the radical 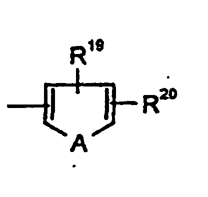 in which
R¹⁹ and R²⁰ are identical or different and each represent hydrogen, fluorine, chlorine, bromine, cyano, nitro, C₁-C₄-alkyl (which is optionally substituted by fluorine and/or chlorine), C₁-C₄-alkoxy (which is optionally substituted by fluorine and/or chlorine), C₁-C₄-alkylthio, C₁-C₄-alkylsulphinyl or C₁-C₄-alkylsulphonyl (each of which is optionally substituted by fluorine and/or chlorine), di-(C₁-C₄-alkyl)-amino-sulphonyl, C₁-C₄-alkoxy-carbonyl or dimethylaminocarbonyl, and
A represents sulphur,
and the sodium, potassium, magnesium, calcium, ammonium, C₁-C₄-alkyl-ammonium, di-(C₁-C₄-alkyl)-ammonium, tri-(C₁-C₄-alkyl)-ammonium, tetra-(C₁-C₄-alkyl)-ammonium, tri-(C₁-C₄-alkyl)-sulphonium, C₅- or C₆-cycloalkyl-ammonium and di-(C₁-C₂-alkyl)-benzyl-ammonium salts thereof.

2. Compounds of the formula (I) according to Claim 1, **characterized in that**
Q¹ represents oxygen,
Q² represents sulphur,
R¹ represents hydrogen, hydroxyl or amino, or represents C₃-C₈-alkylideneamino or represents respectively optionally fluorine-, chlorine-, cyano-, methoxy- or ethoxy-substituted methyl, ethyl, n- or i-propyl, n-, i-, s- or t-butyl, or represents respectively optionally fluorine-, chlorine- or bromine-substituted propenyl, butenyl, propinyl or butinyl, or represents respectively optionally fluorine-, chlorine-, cyano-, methoxy- or ethoxy-substituted methoxy, ethoxy, n- or i-propoxy, n-, i-, s- or t-butoxy, methylamino, ethylamino, n- or i-propylamino, n-, i-, s- or t-butylamino, or represents propenyloxy or butenyloxy, or represents dimethylamino or diethylamino, or represents respectively optionally fluorine-, chlorine-, methyl- and/or ethyl-substituted cyclopropyl, cyclobutyl, cyclopentyl, cyclohexyl, cyclopropylamino, cyclobutylamino, cyclopentylamino, cyclohexylamino, cyclopropylmethyl, cyclobutylmethyl, cyclopentylmethyl or cyclohexylmethyl, or represents respectively optionally fluorine-, chlorine-, methyl-, trifluoromethyl- and/or methoxy-substituted phenyl or benzyl,
R² represents hydrogen, hydroxyl, mercapto, amino, fluorine, chlorine or bromine, or represents respectively optionally fluorine-, chlorine-, cyano-, methoxy- or ethoxy-substituted methyl, ethyl, n- or i-propyl, n-, i-, s- or t-butyl, or represents respectively optionally fluorine-, chlorine- or bromine-substituted propenyl, butenyl, propinyl or butinyl, or represents respectively optionally fluorine-, chlorine-, cyano-, methoxy- or ethoxy-substituted methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, or represents propenyloxy, butenyloxy, propinyloxy, butinyloxy, propenylthio, butenylthio, propinylthio, butinylthio, propenylamino, butenylamino, propinylamino or butinylamino, or represents dimethylamino, diethylamino or dipropylamino, or represents respectively optionally fluorine-, chlorine-, methyl- and/or ethyl-substituted cyclopropyl, cyclobutyl, cyclopentyl, cyclohexyl, cyclopentenyl, cyclohexenyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopropylmethylthio, cyclobutylmethylthio, cyclopentylmethylthio, cyclohexylmethylthio, cyclopropylmethylamino, cyclobutylmethylamino, cyclopentylmethylamino or cyclohexylmethylamino, or represents respectively optionally fluorine-, chlorine-, methyl-, trifluoromethyl- and/or methoxy-substituted phenyl, benzyl, phenoxy, benzyloxy, phenylthio, benzylthio, phenylamino or benzylamino, or
R¹ and R² together represent optionally branched alkanediyl having 3 to 11 carbon atoms, and
R³ represents the radical 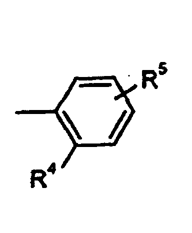 in which
R⁴ represents fluorine, chlorine or bromine, or represents respectively optionally fluorine- and/or chlorine-substituted methyl, ethyl, n- or i-propyl, or represents respectively optionally fluorine-, chlorine-, methoxy- or ethoxy-substituted methoxy, ethoxy, n- or i-propoxy, methylthio, ethylthio, n- or i-propylthio, methylsulphinyl, ethylsulphinyl, n- or i-propylsulphinyl, methylsulphonyl, ethylsulphonyl, n- or i-propylsulphonyl, dimethylaminosulphonyl, diethylaminosulphonyl, N-methoxy-N-methylaminosulphonyl, methoxyaminosulphonyl, phenyl or phenoxy, or represents respectively optionally fluorine-, chlorine-, methoxy- or ethoxy-substituted methoxycarbonyl, ethoxycarbonyl, n- or i-propoxycarbonyl, and
R⁵ represents hydrogen, methyl, ethyl, fluorine, chlorine or bromine;
furthermore,
R³ represents the radical 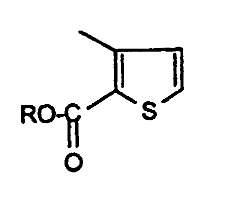 in which
R represents methyl, ethyl, n- or i-propyl.

3. Process for preparing sulphonylaminothiocarbonyl-triazolinones of the formula (I) and salts thereof according to Claim 1, **characterized in that**
(a) triazolinones of the general formula (II) 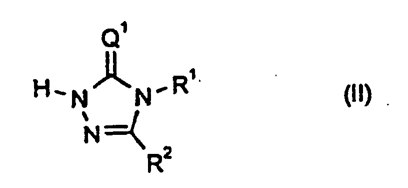 in which
Q¹, R¹ and R² are each as defined in Claim 1,
are reacted with sulphonyl isothiocyanates of the general formula (III)
R³-SO₂-N=C=Q² (III)
in which
Q² and R³ are each as defined in Claim 1,
if appropriate in the presence of a reaction auxiliary and if appropriate in the presence of a diluent,
or
(b) triazolinone derivatives of the general formula (IV) 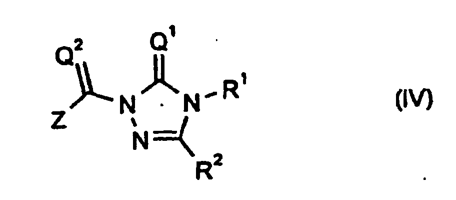 in which
Q¹, Q², R¹ and R² are each as defined in Claim 1 and
Z represents halogen, alkoxy, aryloxy or arylalkoxy,
are reacted with sulphonamides of the general formula (V)
R³-SO₂-NH₂ (V)
in which
R³ is as defined in Claim 1,
if appropriate in the presence of an acid acceptor and if appropriate in the presence of a diluent,
or
(c) triazolinones of the general formula (II) 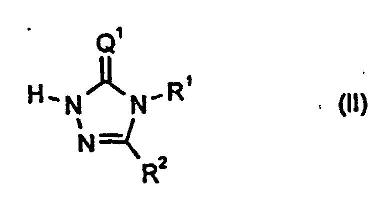 in which
Q¹, R¹ and R² are each as defined in Claim 1,
are reacted with sulphonamide derivatives of the general formula (VI)
R³-SO₂-NH-CQ²-Z (VI)
in which
Q² and R³ are each as defined in Claim 1 and
Z represents halogen, alkoxy, aryloxy or arylalkoxy,
if appropriate in the presence of an acid acceptor and if appropriate in the presence of a diluent,
or
(d) triazolinones of the general formula (II) 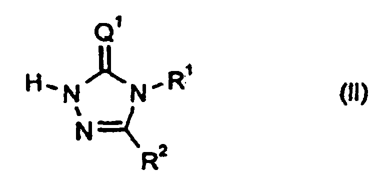 in which
Q¹, R¹ and R² are each as defined in Claim 1,
are reacted with sulphonyl halides of the general formula (VII)
R³-SO₂-X (VII)
in which
R³ is as defined in Claim 1 and
X represents halogen,
and metal thiocyanates of the general formula (VIII)
MQ²CN (VIII)
in which
Q² is as defined in Claim 1 and
M represents an alkali metal or an alkaline earth metal equivalent,
if appropriate in the presence of a reaction auxiliary and if appropriate in the presence of a diluent,
and the compounds of the formula (I) obtained by processes (a), (b), (c) or (d) are, if desired, converted into salts by customary methods.

4. Herbicidal compositions, **characterized by** a content of at least one compound of the formula (I) or a salt thereof according to Claim 1.

5. Use of compounds of the general formula (I) or of salts thereof according to Claim 1 for controlling undesired plant growth.

6. Method for controlling weeds, **characterized in that** compounds of the general formula (I) or salts thereof according to Claim 1 are allowed to act on the weeds or their habitat.

7. Method of preparing herbicidal compositions, **characterized in that** compounds of the general formula (I) or salts thereof according to Claim 1 are mixed with extenders and/or surface-active agents.

8. Triazolinone derivatives of the general formula (IV) 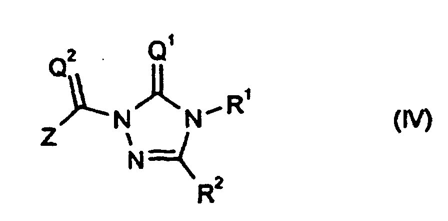 in which
Q¹ represents oxygen,
Q² represents sulphur,
R¹ represents hydrogen or hydroxyl or represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, or represents respectively optionally fluorine-, chlorine- and/or bromine-substituted C₂-C₆-alkenyl or C₂-C₆-alkinyl, or represents optionally fluorine-, chlorine-, cyano-, C₁-C₄-alkoxy- or C₁-C₄-alkoxy-carbonyl-substituted C₁-C₆-alkoxy, or represents C₃-C₆-alkenyloxy, or represents respectively optionally fluorine-, chlorine-, bromine-, cyano- and/or C₁-C₄-alkyl-substituted C₃-C₆-cycloalkyl or C₃-C₆-cycloalkyl-C₁-C₄-alkyl,
R² represents hydrogen, hydroxyl, mercapto, amino, fluorine, chlorine, bromine or iodine, or represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, or represents respectively optionally fluorine-, chlorine- and/or bromine-substituted C₂-C₆-alkenyl or C₂-C₆-alkinyl, or represents respectively optionally fluorine-, chlorine-, cyano-, C₁-C₄-alkoxy- or C₁-C₄-alkoxy-carbonyl-substituted C₁-C₆-alkoxy, C₁-C₆-alkylthio, C₁-C₆-alkylamino or C₁-C₆-alkanoylamino, or represents C₃-C₆-alkenyloxy, C₃-C₆-alkinyloxy, C₃-C₆-alkenylthio, C₃-C₆-alkinylthio, C₃-C₆-alkenylamino or C₃-C₆-alkinylamino, or represents di-(C₁-C₄-alkyl)-amino, or represents respectively optionally methyl- and/or ethyl-substituted aziridino, pyrrolidino, piperidino or morpholino, or represents respectively optionally fluorine-, chlorine-, bromine-, cyano- and/or C₁-C₄-alkyl-substituted C₃-C₆-cycloalkyl, C₅-C₆-cycloalkenyl, C₃-C₆-cycloalkyloxy, C₃-C₆-cycloalkylthio, C₃-C₆-cycloalkylamino, C₃-C₆-cycloalkyl-C₁-C₄-alkyl, C₃-C₆-cycloalkyl-C₁-C₄-alkoxy, C₃-C₆-cycloallcyl-C₁-C₄-alkylthio or C₃-C₆-cycloalkyl-C₁-C₄-alkylamino, or represents respectively optionally fluorine-, chlorine-, bromine-, cyano-, nitro-, C₁-C₄-alkyl-, trifluoromethyl- and/or C₁-C₄-alkoxy-substituted phenyl, phenyl-C₁-C₄-alkyl, phenoxy, phenyl-C₁-C₄-alkoxy, phenylthio, phenyl-C₁-C₄-alkylthio, phenylamino or phenyl-C₁-C₄-alkylamino, or
R¹ and R² together represent optionally branched alkanediyl having 3 to 11 carbon atoms, and
Z represents fluorine, chlorine, bromine, methoxy, ethoxy, benzyloxy or phenoxy.

## Revendications

1. Sulfonylaminothiocarbonyl-triazolinones de formule générale (I) 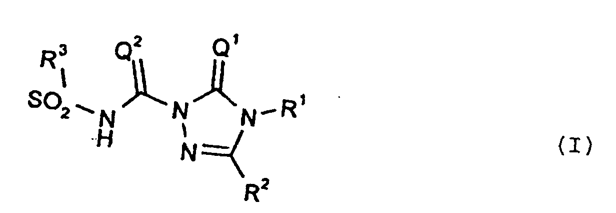 dans laquelle
Q¹ représente l'oxygène,
Q² représente le soufre,
R¹ représente l'hydrogène, un groupe hydroxy, amino, un reste alkylidènamino en C₂ à C₁₀, un reste alkyle en C₁ à C₆ éventuellement substitué par un radical fluoro, chloro, bromo, cyano, alkoxy en C₁ à C₄, (alkyle en C₁ à C₄)-carbonyle ou (alkoxy en C₁ à C₄)-carbonyle, un reste alcényle en C₂ à C₆ ou alcynyle en C₂ à C₆, chacun éventuellement substitué par du fluor, du chlore et/ou du brome, un reste alkoxy en C₁ à C₆, alkylamino en C₁ à C₆ ou alcanoylamino en C₁ à C₆, chacun éventuellement substitué par un radical fluoro, chloro, cyano, alkoxy en C₁ à C₄ ou (alkoxy en C₁ à C₄)-carbonyle, un reste alcényloxy en C₃ à C₆, di-(alkyle en C₁ à C₄)-amino, un reste cycloalkyle en C₃ à C₆, cycloalkylamino en C₃ à C₆ ou (cycloalkyle en C₃ à C₆)-(alkyle en C₁ à C₄) chacun éventuellement substitué par un radical fluoro, chloro, bromo, cyano et/ou alkyle en C₁ à C₄, ou un reste phényle ou phényl-(alkyle en C₁ à C₄), chacun éventuellement substitué par un radical fluoro, chloro, bromo, cyano, nitro, alkyle en C₁ à C₄, trifluorométhyle et/ou alkoxy en C₁ à C₄,
R² représente l'hydrogène, un groupe hydroxy, mercapto, amino, le fluor, le chlore, le brome, l'iode, un reste alkyle en C₁ à C₆ éventuellement substitué par un radical fluoro, chloro, bromo, cyano, alkoxy en C₁ à C₄, (alkyle en C₁ à C₄)-carbonyle ou (alkoxy en C₁ à C₄)-carbonyle, un reste alcényle en C₂ à C₆ ou alcynyle en C₂ à C₆, chacun éventuellement substitué par du fluor, du chlore et/ou du brome, un reste alkoxy en C₁ à C₆, alkylthio en C₁ à C₆, alkylamino en C₁ à C₆ ou alcanoylamino en C₁ à C₆, chacun éventuellement substitué par un radical fluoro, chloro, cyano, alkoxy en C₁ à C₄ ou (alkoxy en C₁ à C₄)-carbonyle, un reste alcényloxy en C₃ à C₆, alcynyloxy en C₃ à C₆, alcénylthio en C₃ à C₆, alcynylthio en C₃ à C₆, alcénylamino en C₃ à C₆ ou alcynylamino en C₃ à C₆, un reste di-(alkyle en C₁ à C₄)-amino, un reste aziridino, pyrrolidino, pipéridino ou morpholino, chacun éventuellement substitué par un radical méthyle et/ou éthyle, un reste cycloalkyle en C₃ à C₆, cycloalcényle en C₅ ou C₆, cycloalkyloxy en C₃ à C₆, cycloalkylthio en C₃ à C₆, cycloalkylamino en C₃ à C₆, (cycloalkyle en C₃ à C₆) - (alkyle en C₁ à C₄) , (cycloalkyle en C₃ à C₆) - (alkoxy en C₁ à C₄) , (cycloalkyle en C₃ à C₆) - (alkylthio en C₁ à C₄) ou (cycloalkyle en C₃ à C₆)-(alkylamino en C₁ à C₄), chacun éventuellement substitué par un radical fluoro, chloro, bromo, cyano et/ou alkyle en C₁ à C₄, ou bien un reste phényle, phényl-(alkyle en C₁ à C₄), phénoxy, phényl-(alkoxy en C₁ à C₄), phénylthio, phényl-(alkylthio en C₁ à C₄), phénylamino ou phényl-(alkylamino en C₁ à C₄), chacun éventuellement substitué par un radical fluoro, chloro, bromo, cyano, nitro, alkyle en C₁ à C₄, trifluorométhyle et/ou alkoxy en C₁ à C₄, ou bien
R¹ et R² forment ensemble un reste alcanediyle éventuellement ramifié ayant 3 à 11 atomes de carbone, et
R³ représente le groupement 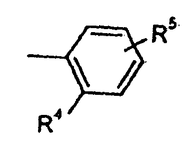 dans lequel
R⁴ et R⁵ sont identiques ou différentes et représentent l'hydrogène, le fluor, le chlore, le brome, l'iode, un groupe nitro, un reste alkyle en C₁ à C₆ (qui est éventuellement substitué par un radical fluoro, chloro, bromo, cyano, carboxy, (alkoxy en C₁ à C₄)-carbonyle, (alkylamino en C₁ à C₄)-carbonyle, di-(alkyle en C₁ à C₄)-aminocarbonyle, hydroxy, alkoxy en C₁ à C₄, formyloxy, (alkyle en C₁ à C₄)-carbonyloxy, (alkoxy en C₁ à C₄)-carbonyloxy, (alkylamino en C₁ à C₄)-carbonyloxy, alkylthio en C₁ à C₄, alkylsulfinyle en C₁ à C₄, alkylsulfonyle en C₁ à C₄, di-(alkyle en C₁ à C₄)-aminosulfonyle, cycloalkyle en C₃ à C₆ ou phényle), un reste alcényle en C₂ à C₆ (qui est éventuellement substitué par un radical fluoro, chloro, bromo, cyano, (alkoxy en C₁ à C₄)-carbonyle, carboxy ou phényle), un reste alcynyle en C₂ à C₆ (qui est éventuellement substitué par un radical fluoro, chloro, bromo, cyano, (alkoxy en C₁ à C₄)-carbonyle, carboxy ou phényle), un reste alkoxy en C₁ à C₄ (qui est éventuellement substitué par un radical fluoro, chloro, bromo, cyano, carboxy, (alkoxy en C₁ à C₄)-carbonyle, alkoxy en C₁ à C₄, alkylthio en C₁ à C₄, alkylsulfinyle en C₁ à C₄ ou alkylsulfonyle en C₁ à C₄), un reste alkylthio en C₁ à C₄ (qui est éventuellement substitué par un radical fluoro, chloro, bromo, cyano, carboxy, (alkoxy en C₁ à C₄)-carbonyle, alkylthio en C₁ à C₄, alkylsulfinyle en C₁ à C₄ ou alkylsulfonyle en C₁ à C₄), un reste alcényloxy en C₂ à C₆ (qui est éventuellement substitué par un radical fluoro, chloro, bromo, cyano ou (alkoxy en C₁ à C₄)-carbonyle, un reste alcénylthio en C₂ à C₆ (qui est éventuellement substitué par un radical fluoro, chloro, bromo, cyano, nitro, alkylthio en C₁ à C₃ ou (alkoxy en C₁ à C₄)-carbonyle), un reste alcynyloxy en C₃ à C₆, alcynylthio en C₃ à C₆ ou le reste -S(O)ₚ-R⁶ dans lequel
p représente le nombre 1 ou 2 et
R⁶ est un reste alkyle en C₁ à C₄ (qui est éventuellement substitué par un radical fluoro, chloro, bromo, cyano ou (alkoxy en C₁ à C₄)-carbonyle, un reste alcényle en C₃ à C₆, alcynyle en C₃ à C₆, alkoxy en C₁ à C₄, (alkoxy en C₁ à C₄)-(alkyle en C₁ à C₄)-amino, alkylamino en C₁ à C₄, di-(alkyle en C₁ à C₄)-amino, phényle, ou le reste -NHOR₇, dans lequel
R⁷ est un reste alkyle en C₁ à C₁₂ (qui est éventuellement substitué par un radical fluoro, chloro, cyano, alkoxy en C₁ à C₄, alkylthio en C₁ à C₄, alkylsulfinyle en C₁ à C₄, alkylsulfonyle en C₁ à C₄, (alkyle en C₁ à C₄)-carbonyle, (alkoxy en C₁ à C₄)-carbonyle, (alkylamino en C₁ à C₄)-carbonyle ou di-(alkyle en C₁ à C₄)-aminocarbonyle), un reste alcényle en C₃ à C₆ (qui est éventuellement substitué par du fluor, du chlore ou du brome), un reste alcynyle en C₃ à C₆, cycloalkyle en C₃ à C₆, (cycloalkyle en C₃ à C₆)-alkyle en C₁ ou C₂, phényl-(alkyle en C₁ ou C₂) (qui est éventuellement substitué par un radical fluoro, chloro, nitro, cyano, alkyle en C₁ à C₄, alkoxy en C₁ à C₄ ou (alkoxy en C₁ à C₄)-carbonyle), un reste benzhydrile ou un reste phényle (qui est éventuellement substitué par un radical fluoro, chloro, nitro, cyano, alkyle en C₁ à C₄, trifluorométhyle, alkoxy en C₁ à C₄, fluoralkoxy en C₁ ou C₂, alkylthio en C₁ à C₄, trifluorométhylthio ou (alkoxy en C₁ à C₄) -carbonyle,
R⁴ et/ou R⁵ représentent en outre un reste phényle ou un reste phénoxy, un reste (alkyle en C₁ à C₄)-carbonylamino, (alkoxy en C₁ à C₄)-carbonylamino, (alkyle en C₁ à C₄)-aminocarbonylamino, di-(alkyle en C₁ à C₄)-aminocarbonylamino ou le reste -CO-R⁸, dans lequel
R⁸ représente l'hydrogène, un reste alkyle en C₁ à C₆, cycloalkyle en C₃ à C₆, alkoxy en C₁ à C₆, cycloalkoxy en C₃ à C₆, alcényloxy en C₃ à C₆, alkylthio en C₁ à C₄, alkylamino en C₁ à C₄, alkoxyamino en C₁ à C₄, (alkoxy en C₁ à C₄)-(alkylamino en C₁ à C₄) ou di-(alkyle en C₁ à C₄)-amino) qui sont éventuellement substitués par du fluor et/ou du chlore,
R⁴ et/ou R⁵ représentent en outre un reste triméthylsilyle, thiazolinyle, alkylsulfonyloxy en C₁ à C₄, di-(alkyle en C₁ à C₄)-aminosulfonylamino ou le reste -CH=N-R⁹, dans lequel
R⁹ désigne un reste alkyle en C₁ à C₆ éventuellement substitué par un radical fluoro, chloro, cyano, carboxy, alkoxy en C₁ à C₄, alkylthio en C₁ à C₄, alkylsulfinyle en C₁ à C₄ ou alkylsulfonyle en C₁ à C₄, un reste benzyle éventuellement substitué par du fluor ou du chlore, un reste alcényle en C₃ à C₆ ou alcynyle en C₃ à C₆ éventuellement substitué par du fluor ou du chlore, un reste phényle éventuellement substitué par un radical fluoro, chloro, bromo, alkyle en C₁ à C₄, alkoxy en C₁ à C₄, trifluorométhyle, trifluorométhoxy ou trifluorométhylthio, un reste alkoxy en C₁ à C₆ éventuellement substitué par du fluor et/ou du chlore, un reste alcényloxy en C₃ à C₆, alcynyloxy en C₃ à C₆ ou benzyloxy, un reste amino, alkylamino en C₁ à C₄, di-(alkyle en C₁ à C₄)-amino, phénylamino, (alkyle en C₁ à C₄)-carbonylamino, (alkoxy en C₁ à C₄)-carbonylamino, alkylsulfonylamino en C₁ à C₄ ou un reste phénylsulfonylamino éventuellement substitué par un radical fluoro, chloro, bromo ou méthyle, ou bien,
en outre
R³ représente le reste 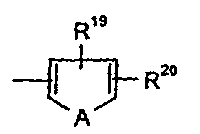 dans lequel
R¹⁹ et R²⁰ sont identiques ou différentes et représentent l'hydrogène, le fluor, le chlore, le brome, un groupe cyano, nitro, un reste alkyle en C₁ à C₄ (qui est éventuellement substitué par du fluor et/ou du chlore), un reste alkoxy en C₁ à C₄ (qui est éventuellement substitué par du fluor et/ou du chlore), un reste alkylthio en C₁ à C₄, alkylsulfinyle en C₁ à C₄ ou alkylsulfonyle en C₁ à C₄ (chacun étant éventuellement substitué par du fluor et/ou du chlore), un reste di-(alkyle en C₁ à C₄)-aminosulfonyle, (alkoxy en C₁ à C₄)-carbonyle ou diméthylaminocarbonyle, et
A représente le soufre,
ainsi que leurs sels de sodium, de potassium, de magnésium, de calcium, d'ammonium, de (alkyle en C₁ à C₄)-ammonium, de di-(alkyle en C₁ à C₄)-ammonium, de tri-(alkyle en C₁ à C₄)-ammonium, de tétra-(alkyle en C₁ à C₄)-ammonium, de tri-(alkyle en C₁ à C₄)-sulfonium, de (cycloalkyle en C₅ ou C₆)-ammonium et de di-(alkyle en C₁ ou C₂)-benzylammonium.

2. Composés de formule (I) suivant la revendication 1, **caractérisés en ce que**, dans cette formule
Q¹ représente l'oxygène,
Q² représente le soufre,
R¹ représente l'hydrogène, un groupe hydroxy, amino, un reste alkylidènamino en C₃ à C₈, un reste méthyle, éthyle, n-propyle, isopropyle, n-butyle, isobutyle, sec.-butyle ou tertiobutyle, chacun éventuellement substitué par un radical fluoro, chloro, cyano, méthoxy ou éthoxy, un reste propényle, butényle, propinyle ou butynyle, chacun éventuellement substitué par du fluor, du chlore ou du brome, un reste méthoxy, éthoxy, n-propoxy, isopropoxy, n-butoxy, isobutoxy, sec.-butoxy, tertiobutoxy, méthylamino, éthylamino, n-propylamino, isopropylamino, n-butylamino, isobutylamino, sec.-butylamino ou tertiobutylamino, chacun éventuellement substitué par un radical fluoro, chloro, cyano, méthoxy, ou éthoxy, un reste propényloxy ou butényloxy, un reste diméthylamino ou diéthylamino, un reste cyclopropyle, cyclobutyle, cyclopentyle, cyclohexyle, cyclopropylamino, cyclobutylamino, cyclopentylamino, cyclohexylamino, cyclopropylméthyle, cyclobutylméthyle, cyclopentylméthyle ou cyclohexylméthyle, chacun éventuellement substitué par un radical fluoro, chloro, méthyle et/ou éthyle, un reste phényle ou benzyle, chacun éventuellement substitué par un radical fluoro, chloro, méthyle, trifluorométhyle et/ou méthoxy,
R² représente l'hydrogène, un groupe hydroxy, mercapto, amino, le fluor, le chlore, le brome, un reste méthyle, éthyle, n-propyle, isopropyle, n-butyle, isobutyle, sec.-butyle ou tertiobutyle, chacun éventuellement substitué par un radical fluoro, chloro, cyano, méthoxy ou éthoxy, un reste propényle, butényle, propynyle ou butynyle, chacun éventuellement substitué par du fluor, du chlore ou du brome, un reste méthoxy, éthoxy, n-propoxy, isopropoxy, n-butoxy, isobutoxy, sec.-butoxy, tertiobutoxy, méthylthio, éthylthio, n-propylthio, isopropylthio, n-butylthio, isobutylthio, sec.-butylthio, tertiobutylthio, méthylamino, éthylamino, n-propylamino, isopropylamino, n-butylamino, isobutylamino, sec.-butylamino ou tertiobutylamino, chacune éventuellement substituée par un radical fluoro, chloro, cyano, méthoxy ou éthoxy, un reste propényloxy, butényloxy, propynyloxy, butynyloxy, propénylthio, buténylthio, propinylthio, butinylthio, propénylamino, buténylamino, propinylamino ou butinylamino, un reste diméthylamino, diéthylamino ou dipropylamino, un reste cyclopropyle, cyclobutyle, cyclopentyle, cyclohexyle, cyclopentényle, cyclohexényle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylméthyle, cyclobutylméthyle, cyclopentylméthyle, cyclohexylméthyle, cyclopropylméthoxy, cyclobutylméthoxy, cyclopentylméthoxy, cyclohexylméthoxy, cyclopropylméthylthio, cyclobutylméthylthio, cyclopentylméthylthio, cyclohexylméthylthio, cyclopropylméthylamino, cyclobutylméthylamino, cyclopentylméthylamino ou cyclohexylméthylamino, chacun éventuellement substitué par un radical fluoro, chloro, méthyle et/ou éthyle, ou bien un reste phényle, benzyle, phénoxy, benzyloxy, phénylthio, benzylthio, phénylamino ou benzylamino, chacun éventuellement substitué par un radical fluoro, chloro, méthyle, trifluorométhyle et/ou méthoxy, ou bien
R¹ et R² forment ensemble un reste alcanediyle éventuellement ramifié ayant 3 à 11 atomes de carbone, et
R³ représente le reste 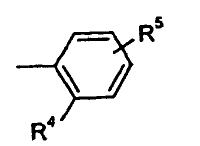
dans lequel
R⁴ représente le fluor, le chlore, le brome, un reste méthyle, éthyle, n-propyle ou isopropyle, chacun éventuellement substitué par du fluor et/ou du chlore, un reste méthoxy, éthoxy, n-propoxy ou isopropoxy, méthylthio, éthylthio, n-propylthio ou isopropylthio, méthylsulfinyle, éthylsulfinyle, n-propylsulfinyle ou isopropylsulfinyle, méthylsulfonyle, éthylsulfonyle, n-propylsulfonyle, isopropylsulfonyle, diméthylaminosulfonyle, diéthylaminosulfonyle, N-méthoxy-N-méthylaminosulfonyle, méthoxyaminosulfonyle, phényle, phénoxy, chacun éventuellement substitué par un radical fluoro, chloro, méthoxy ou éthoxy, ou un reste méthoxycarbonyle, éthoxycarbonyle, n-propoxycarbonyle ou isopropoxycarbonyle, chacun éventuellement substitué par un radical fluoro, chloro, méthoxy ou éthoxy, et
R⁵ représente l'hydrogène, un reste méthyle, éthyle, le fluor, le chlore ou le brome ; en outre
R³ représente le reste 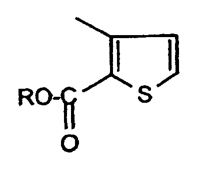
dans lequel
R est un reste méthyle, éthyle, n-propyle ou isopropyle.

3. Procédé de production de sulfonylaminothiocarbonyltriazolinones de formule (I) et de leurs sels suivant la revendication 1, **caractérisé en ce que**
(a) on fait réagir des triazolinones de formule générale (II) 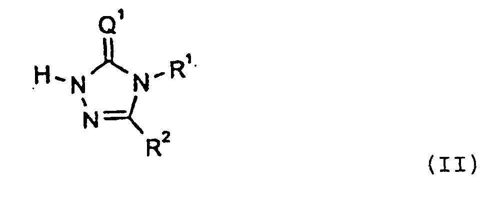 dans laquelle
Q¹, R¹ et R² ont la définition indiquée dans la revendication 1,
avec des sulfonylisothiocyanates de formule générale (III)
R³-SO₂-N=C=Q² (III)
dans laquelle
Q² et R³ ont la définition indiquée dans la revendication 1,
éventuellement en présence d'un auxiliaire de réaction et, le cas échéant, en présence d'un diluant,
ou bien
(b) on fait réagir des dérivés de triazolinone de formule générale (IV) 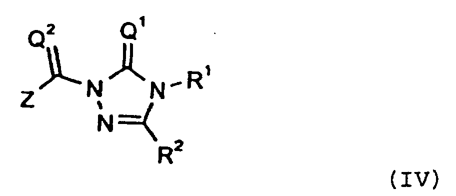 dans laquelle
Q¹, Q² et R¹ et R2 ont la définition indiquée dans la revendication 1 et
Z est un halogène, un reste alkoxy, aryloxy ou arylalkoxy,
avec des amides d'acide sulfonique de formule générale (V)
R³-SO₂-NH₂ (V)
dans laquelle
R³ a la définition indiquée dans la revendication 1, éventuellement en présence d'un accepteur d'acide et, le cas d'échant, en présence d'un diluant,
ou bien
(c) on fait réagir des triazolinones de formule générale (II) 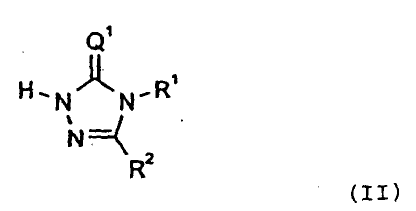 dans laquelle
Q¹, R¹ et R² ont la définition indiquée dans la revendication 1,
avec des dérivés d'amides d'acides sulfoniques de formule générale (VI)
R³-SO₂-NH-CQ²-Z (VI)
dans laquelle
Q² et R³ ont la définition indiquée dans la revendication 1 et
Z représente un halogène, un reste alkoxy, aryloxy ou arylalkoxy,
éventuellement en présence d'un accepteur d'acide et, le cas échéant, en présence d'un diluant,
ou bien
(d) on fait réagir des triazolinones de formule générale (II) 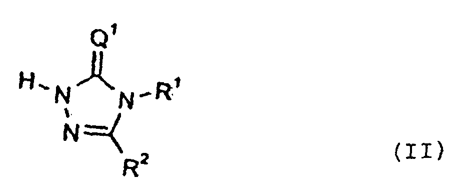 dans laquelle
Q¹, R¹ et R² ont la définition indiquée dans la revendication 1,
avec des halogénures d'acides sulfoniques de formule générale (VII)
R³-SO₂-X (VII)
dans laquelle
R³ a la définition indiquée dans la revendication 1 et X est un halogène,
et des thiocyanates métalliques de formule générale (VIII)
MQ²CN (VIII)
dans laquelle
Q² a la définition indiquée dans la revendication 1 et
M est un métal alcalin ou un équivalent de métal alcalinoterreux,
éventuellement en présence d'un auxiliaire de réaction et, le cas échéant, en présence d'un diluant,
et on transforme éventuellement en sels, par des modes opératoires classiques, les composés de formule (I) obtenus selon le procédé (a), (b), (c) ou (d).

4. Compositions herbicides, **caractérisées par** une teneur en au moins un composé de formule (I) ou en l'un de leurs sels suivant la revendication 1.

5. Utilisation de composés de formule générale (I) ou de leurs sels suivant la revendication 1 pour la lutte contre une végétation indésirable.

6. Procédé de lutte contre des mauvaises herbes, **caractérisé en ce qu'**on fait agir des composés de formule générale (I) ou leurs sels suivant la revendication 1 sur les mauvaises herbes ou sur leur milieu.

7. Procédé de préparation de compositions herbicides, **caractérisé en ce qu'**on mélange des composés de formule générale (I) ou leurs sels suivant la revendication 1 avec des diluants et/ou des agents tensioactifs.

8. Dérivés de triazolinone de formule (IV) 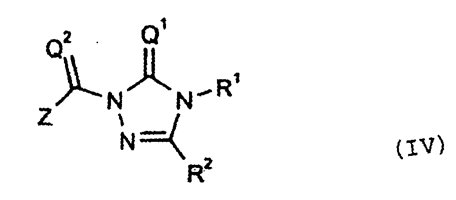 dans laquelle
Q¹ représente l'oxygène,
Q² représente le soufre,
R¹ représente l'hydrogène, un groupe hydroxy, un reste alkyle en C₁ à C₆ éventuellement substitué par un radical fluoro, chloro, bromo, cyano, alkoxy en C₁ à C₄, (alkyle en C₁ à C₄)-carbonyle ou (alkoxy en C₁ à C₄)-carbonyle, un reste alcényle en C₂ à C₆ ou alcynyle en C₂ à C₆, chacun éventuellement substitué par du fluor, du chlore et/ou du brome, un reste alkoxy en C₁ à C₆ éventuellement substitué par un radical fluoro, chloro, cyano, alkoxy en C₁ à C₄ ou (alkoxy en C₁ à C₄)-carbonyle, un reste alcényloxy en C₃ à C₆, un reste cycloalkyle en C₃ à C₆ ou (cycloalkyle en C₃ à C₆)-(alkyle en C₁ à C₆) chacun éventuellement substitué par un radical fluoro, chloro, bromo, cyano et/ou alkyle en C₁ à C₄,
R² représente l'hydrogène, un groupe hydroxy, mercapto, amino, le fluor, le chlore, le brome, l'iode, un reste alkyle en C₁ à C₆ éventuellement substitué par un radical fluoro, chloro, bromo, cyano, alkoxy en C₁ à C₄, (alkyle en C₁ à C₄)-carbonyle ou (alkoxy en C₁ à C₄)-carbonyle, un reste alcényle en C₂ à C₆ ou alcynyle en C₂ à C₆, chacun éventuellement substitué par du fluor, du chlore et/ou du brome, un reste alkoxy en C₁ à C₆, alkylthio en C₁ à C₆, alkylamino en C₁ à C₆ ou alcanoylamino en C₁ à C₆, chacun éventuellement substitué par un radical fluoro, chloro, cyano, alkoxy en C₁ à C₄ ou (alkoxy en C₁ à C₄)-carbonyle, un reste alcényloxy en C₃ à C₆, alcynyloxy en C₃ à C₆, alcénylthio en C₃ à C₆, alcynylthio en C₃ à C₆, alcénylamino en C₃ à C₆ ou alcynylamino en C₃ à C₆, un reste di-(alkyle en C₁ à C₄)-amino, un reste aziridino, pyrrolidino, pipéridino ou morpholino, chacun éventuellement substitué par un radical méthyle et/ou éthyle, un reste cycloalkyle en C₃ à C₆, cycloalcényle en C₅ ou C₆, cycloalkyloxy en C₃ à C₆, cycloalkylthio en C₃ à C₆, cycloaklylamino en C₃ à C₆, (cycloalkyle en C₃ à C₆)-(alkyle en C₁ à C₄), (cycloalkyle en C₃ à C₆)-(alkoxy en C₁ à C₄), (cycloalkyle en C₃ à C₆)-(alkylthio en C₁ à C₄) ou (cycloalkyle en C₃ à C₆)-(alkylamino en C₁ à C₄), chacun éventuellement substitué par un radical fluoro, chloro, bromo, cyano et/ou alkyle en C₁ à C₄, ou bien un reste phényle, phényl-(alkyle en C₁ à C₄), phénoxy, phényl-(alkoxy en C₁ à C₄), phénylthio, phényl-alkylthio en C₁ à C₄, phénylamino ou phényl-(alkylamino en C₁ à C₄), chacun éventuellement substitué par un radical fluoro, chloro, bromo, cyano, nitro, alkyle en C₁ à C₄, trifluorométhyle et/ou alkoxy en C₁ à C₄,
ou bien
R¹ et R² forment ensemble un reste alcanediyle éventuellement ramifié ayant 3 à 11 atomes de carbone et
Z représente le fluor, le chlore, le brome, un reste méthoxy, éthoxy, benzyloxy ou phénoxy.
